# EUROPEAN PATENT SPECIFICATION

(11) **EP 3 222 139 B1**
(45) Date of publication and mention of the grant of the patent: **19.05.2021**
(21) Application number: 15860466.0
(22) Date of filing: 12.11.2015
(51) Int. Cl.: A01K 13/00, A61K 9/00, A61K 47/12, A61K 47/34

(54) **TEAT PACK MATERIAL FOR LACTATING LIVESTOCK, KIT FOR FORMING TEAT PACK FOR LACTATING LIVESTOCK, AND METHOD FOR PREVENTING MASTITIS IN LACTATING LIVESTOCK**
ZITZENPACKUNSMATERIAL FÜR MILCHVIEH, BAUSATZ ZUR HERSTELLUNG EINER ZITZENPACKUNG FÜR MILCHVIEH UND VERFAHREN ZUR VERHÜTUNG VON MASTITIS BEI MILCHVIEH
MATÉRIAU POUR BLOC-TRAYEUR POUR BÉTAIL PRODUCTEUR DE LAIT, KIT DE FORMATION POUR BLOC-TRAYEUR POUR BÉTAIL PRODUCTEUR DE LAIT, ET PROCÉDÉ POUR EMPÊCHER LA MAMMITE CHEZ LE BÉTAIL PRODUCTEUR DE LAIT

(30) Priority: 17.11.2014 JP 2014232988; 21.05.2015 JP 2015103455; 22.05.2015 JP 2015104432
(43) Date of publication of application: 27.09.2017
(73) Proprietor: Tokuyama Corporation, Shunan-shi, Yamaguchi-ken 745-8648 (JP)
(72) Inventor: KONDO, Hitoshi, Shunan-shi, Yamaguchi 745-8648 (JP); INUI, Yoji, Shunan-shi, Yamaguchi 745-8648 (JP); KAZAMA, Hideki, Shunan-shi, Yamaguchi 745-8648 (JP)
(74) Representative: HGF
(86) International application number: PCT/JP2015/081905
(87) International publication number: WO 2016/080286

(56) References cited:
- JP-A- 2000 041 529
- JP-A- 2006 050 911
- JP-A- 2014 168 451
- US-A- 4 113 854
- US-A- 4 113 854
- US-A- 4 716 180

## Description

### TECHNICAL FIELD

The present invention relates to a teat pack material for lactating livestock, a kit for forming a teat pack for lactating livestock, and a method for preventing mastitis in lactating livestock.

Hereinafter, livestock that produce dairy milk or processed milk such as dairy cattle, goats, and sheep may be collectively referred to as "lactating livestock". Further, the protective film for covering the entirety of the teat (nipple) of lactating livestock to prevent infections such as mastitis may be referred to as a "teat pack for lactating livestock", and the material for forming the "teat pack for lactating livestock" may be referred to as a "teat pack material for lactating livestock".

### BACKGROUND ART

US 4,113,854 A discloses a method for the prophylactic treatment of mastitis in milk-producing animals. The method comprises dipping the teats of the animal into a composition comprising a film-forming polymer latex and a water soluble polymer thickening agent in an aqueous medium. The method uses a coagulant such as polyox WSR coagulant (ethylene oxide polymer) and a para-amino benzoic acid as sunscreen. The ethylene oxide polymer is used as a thickening agent.

JP 2006 050911 A discloses a teat pack and a method for protecting teat and method for applying teat pack. The method uses a composition comprising alginate.

A typical lactating livestock is dairy cattle, thus, the invention is explained below with regards to dairy cattle. Mastitis is a serious disease in dairy cattle. Mastitis is an infectious disease caused by pathogen or other microbes entering from the teat canals to the inside of the mammary, becoming established and proliferating, but the mechanism by which this occurs is complicated, and thus, mastitis is a disease which has still not been eradicated and continues to inflict serious economic losses upon the dairy industry. Mastitis has various forms and symptoms, but the discovery of mastitis with clinical symptoms such as redness, pain, swelling, or fever of the mammary, or the appearance of coagulated milk within the milk is easy, and mastitis tends to decrease worldwide as antibiotic treatment spreads. However, despite the fact that so-called sub-clinical mastitis in which these clinical symptoms are not exhibited, but abnormalities such as an increase in the number of somatic cells are found when examining the milk, inflicts serious economic losses such as a reduction in milk production and milk quality, the control thereof has progressed slowly.

To prevent mastitis, the following important measures referred to as the "5 points" are currently recommended worldwide.
(1) Hygienic measures of milking facilities and the like including the inspection and maintenance of the milkers,
(2) Disinfection of the teat,
(3) Treatment of clinical mastitis,
(4) Dry cow therapy (DCT), and
(5) Culling of problem cattle (Non-Patent Documents 1, 2, and 3).

Among the aforementioned proposed measures, teat disinfection of the dairy cattle is one of the most important preventative measures for preventing mastitis. A teat disinfectant developed and implemented in the UK in 1952 by Dodd, et al., has been used since about 1965 as a part of the milk quality improvement project in Japan and today has a coverage of 40%.

The teat disinfection which is currently implemented is a method (the so-called post-dipping method) for immersing the teat in a dipping agent (sterilization and disinfection aqueous solution) after milking, and mastitis is prevented by sterilizing and disinfecting the mastitis-causing pathogen adhering to the teat skin surface, and furthermore, using a humectant to improve the condition of the teat skin such as cracking, and inhibiting the proliferation of pathogen on the teat surface, and to date, numerous post-dipping agents have been proposed (for example, Patent Document 1 and Patent Document 2), and many of these products are available on the market. However, in testing conducted by the National Institute for Research in Dairying, while new infections in 12 months were reduced by 50% in the group of dairy cattle which underwent post dipping, this was only a 14% reduction from the entirety of the mammaries that had already been infected. It is reported that sub-clinical infections by existing pathogen persist. Namely, while the post-dipping reduces the rate of new infections by contagious mastitis-causing pathogen, the effect which was hoped for cannot be anticipated with respect to the control effect for the causative pathogen of so-called environmental mastitis.

In short, by only disinfecting (post-dipping) after the milking, the effect duration (for example, when the teat touched the cow bedding, after use, 1 to 2 hours until the liquid medicine is removed from the teat and the effect is lost) is relatively short, thus, the sterilization effect is lost until the following milking, and accordingly, there was a limit to the effect on the causative pathogen in the environment.

Meanwhile, as a means for protecting the teat of the dairy cattle from the mastitis-causing pathogen, techniques for protecting the entirety of the teat of the dairy cattle and preventing pathogen from entering the teat canal to inside the mammary have been proposed.

For example, Patent Document 3 discloses "a method for preventing mastitis in a milk cow characterized in physically preventing mastitis-causing bacterial infection by immersing a teat in a teat-sealing agent and maintaining the teat in a state in which a film forms which closes the teat canal in the teat for the initial about two to nine days in the dry period and about two to nine days before the calving of the milk cow, the milk cow being easily infected with mastitis during the dry period" (Claim 1). Furthermore, Patent Document 3 describes teat-sealing agents that are obtained by dissolving a rubber material selected from urethane rubber, latex rubber, butadiene resin, polyvinyl alcohol, liquid butyl rubber, liquid rubber, natural rubber, butyl rubber, nitrile rubber, chloroprene rubber, vinyl acetate rubber, and the like in a solvent, such as a freon substitute such as tetrahydrofuran, acetonitrile, trichloroethane, trichlorethylene, and methylene chloride and an aromatic compound such as toluene and xylene at a concentration of 5 to 15%, as a papilla-sealing agent in which the teat canal is immersed.

However, the rubber material of Patent Document 3 requires time until the solvent volatilizes and forms the pack, thus, when the cattle lays down and rests after the applying of the material and before the formation of the pack, there was the problem that the liquid dissipated and the pack did not form. Further, when the rubber material becomes dry, harmful organic solvents such as acetonitrile and toluene volatilize, thus, there was the risk of harming the health of humans or the dairy cattle.

Further, Patent Document 4 discloses "the teat pack contains at least water, a calcium salt and an alginate salt, and also exhibits a 5,000-1,500,000 mPa·sec viscosity before gelation", and furthermore, Patent Document 4 proposes that a teat can be protected from mastitis-causing pathogen and the like for a long period of time by blending an antimicrobial such as iodine in the teat pack.

However, in the teat pack described in Patent Document 4, it is explained that the paste obtained by mixing a powder component comprising alginate salt and calcium sulfate salt with a solvent such as water immediately before use is used by applying it to the teat. Calcium sulfate is insoluble in water, that is, it is a hydrophobic component, and it is very difficult to uniformly disperse in a hydrophilic solvent such as water, thus, in order to obtain the desired dispersability, not only does it require a great deal of labor and skill for the technician, but there was the problem that the powder component becomes scattered when kneading.

Further, in Patent Document 4, the aforementioned problem is mitigated by dispersing the component comprising the powdered alginate salt and calcium sulfate salt in a hydrophilic solvent such as glycerin or propylene glycol beforehand, but even in this case, it is difficult to disperse calcium sulfate which has a hydrophobic surface in a hydrophilic solvent, thus, sedimentation and separation occurs over time. As a result, in some cases, the dispersability was impaired during use (when kneading with water), the kneading takes time and air bubbles also become mixed therein so that the uniformity and the curability of the teat pack which can be obtained decreases, the adhesiveness to the teat deteriorates and it becomes easy to peel the teat pack from the teat.

Patent Document 5 discloses a method in which the teat of a lactating animal is immersed in a composition comprising a polymer latex and a water-soluble polymer thickening agent in an aqueous dispersion medium for the preventative treatment of mastitis in a lactating animal. Latex is a dispersion of the polymer compound, and if the dispersion is immersed on the teat of lactating livestock, a film is gradually formed over time, and as a result, the teat is protected. The method for protecting the teat using the composition comprising this kind of latex is considered to be a useful technique from the simplicity of the operating method.

The present inventors examined in detail from various aspects the materials constituting the invention described in Patent Document 5 and the practical application. As a result, it is understood that the prior art described in Patent Document 5 has defects to be improves as exemplified below.
(a) Latexes of styrene / butadiene, acrylic polymer and acrylic copolymer have been described as polymers constituting the film-forming polymer latex. Furthermore, latexes of ethyl acrylate / methyl methacrylate copolymer, methyl methacrylate / butyl acrylate copolymer and styrene / butadiene copolymer have been described as preferable film-forming polymer latexes. Latexes of ethyl acrylate / methyl methacrylate copolymer have been described as particularly preferable film-forming polymer latexes (Column 4, lines 1-11). Namely, a natural rubber and a synthetic rubber latex have not been described or suggested. Therefore, the excellent physical properties such as elasticity, abrasion resistance, aging resistance, oil resistance, weatherability usually possessed by natural rubber and synthetic rubber are not utilized, thus, the width of the selection of the material is remarkably narrow.
(b) As water-soluble polymer thickening agents, natural polysaccharides such as guar gum and gum arabic, or a synthetic water-soluble polymers such as modified (semisynthetic) polymers derived from cellulose or poly(vinylpyrrolidone) have been described (column 2, lines 34 to 58, etc). Water-soluble polymer thickening agents are commonly used to adjust the viscosity of latex, but the mechanism thereof is based on the formation of a three-dimensional network structure, and it takes more time to form the film compared to the film formation due to the rapid coagulation by the addition of an electrolyte (coagulant). Column 5, lines 1-3 of Patent Document 5 describes "the compositions dry to a uniform protective film in approximately 20 minutes or less".
(c) The teat of lactating livestock is not always maintained in clean conditions, and specifically, in the case of the milking period, it is often the case that the teat is covered with fats and oils on the teat skin or is covered with milk (hereinafter, the state in which the teat is covered with fats and oils, or, is covered with milk will be referred to as the "teat coated with oil and fat"). Even when trying to protect the teat coated with oil and fat using a well-known latex, the coatability onto the teat was poor, and satisfactory results were not obtained for adhesiveness. After removing the oil film of the teat coated with oil and fat, the teat may be protected using latex, but the operation becomes complicated.
(d) The four teats of normal healthy dairy cattle are adjacent to each other in a 2 by 2 arrangement, and goats and sheep have two teats. When protecting the teat of lactating livestock, it is usually necessary to simultaneously protect all of the teats. However, when protecting the adjacent four or two teats, there are numerous cases when the adjacent packs adhere to each other at the time of formation or after formation of the teat pack, when lactating livestock is lying down and resting. If adhesion occurs, the peeling of the pack occurs easily, and as a result, the teat cannot be protected properly. Such a phenomenon occurs remarkably when a latex material, specifically, a latex material comprising a rubber-based polymer is used as the test pack material.
(e) Column 3, lines 19-24 describes "the protective film must be removed from the teat prior to milking. Therefore, it is desirable that the protective film be sufficiently water sensitive so as to be removed by the farmer during his normal pre-milking routine, e.g., by using plain water and a wash cloth". However, it is desirable to simply remove by only a manual operation without using water and a cloth for washing.

Patent Document 1: Japanese Unexamined Patent Application, Publication No. H8-175989
Patent Document 2: Japanese Unexamined Patent Application, Publication No. H11-155404
Patent Document 3: Japanese Unexamined Patent Application, Publication No. 2000-41529
Patent Document 4: Japanese Unexamined Patent Application, Publication No. 2006-50911
Patent Document 5: US Patent Application Publication No. 4113854

Non-Patent Document 1: Livestock Dictionary Editorial Board Representative Hiroshi Nagasawa, February 20, 1996, Livestock Dictionary, Yokendo Co., Ltd
Non-Patent Document 2: Encyclopedia of Physiology, Breeding Technology and Environmental Management, March 31, 2011, Rural Culture Association Japan
Non-Patent Document 3: Eds. Kodansha Scientific Ltd., Shinpen Chikusan Handbook, September 10, 2006, Kodansha Ltd.

### DISCLOSURE OF THE INVENTION

### Problems to be Solved by the Invention

The aforementioned prior art was comprehensively examined, and as a result, the first problem to be solved by the invention is to provide a teat pack for lactating livestock for coating the entire surface of the teat of lactating livestock, excellent in adhesiveness, elasticity, abrasion resistance, durability, aging resistance, oil resistance, weatherability, and breast shape followability, and for preventing lactating livestock from developing mastitis, and a material and a method which instantaneously and easily forms the teat pack for lactating livestock on the entire surface of the teat of lactating livestock.

The second problem to be solved by the invention is to provide a material and a method for instantaneously and easily forming the teat pack for lactating livestock excellent in adhesiveness, elasticity, abrasion resistance, durability, aging resistance, oil resistance, weatherability, etc., on the entire surface of the teat of lactating livestock for preventing lactating livestock from developing mastitis even in the teat coated with oil and fat.

The third problem to be solved by the invention is to provide the material and method for preventing the adjacent packs from adhering to each other even if lactating livestock in which teat packs were formed on all of the teats of which there are 2 or 4 adjacent to each other of lactating livestock lays down and rests in a barn or a ranch, etc.

The fourth problem to be solved by the invention is to provide the material and method which makes it possible for the teat pack closely adhered to the teat to be quickly removed without using any additional facility, material, etc., when milking again or in case of emergency.

The fifth problem to be solved by the invention is to provide the teat pack material for lactating livestock which does not deteriorate even in a cold climate.
Further problems to be solved by the invention and the advantages will be clarified below.

### Means for Solving the Problems

First, materials for solving the aforementioned problems were considered based on theories and demonstration tests in the laboratory. As a result, a polymer latex was selected as a base having excellent adhesiveness, elasticity, abrasion resistance, durability, aging resistance, oil resistance, and weatherability, etc., and instantaneously forming the teat pack for lactating livestock for preventing lactating livestock from developing mastitis, on the entire surface of the teat of lactating livestock. Then, a coagulant for rapidly coagulating a dispersoid constituting the polymer latex to form the teat pack was selected. Each of the polymer latex and the coagulant was adopted as the essential component for forming the respective teat packs. Next, a surfactant was adopted as a material which ensures the formed teat pack maintains resistance and stability to a teat coated with oil and fat as well. Furthermore, a release agent was adopted as a material which makes it possible for the teat pack to be quickly removed without using any additional facility, material, etc. when milking again or in case of emergency. Furthermore, an antifreezing agent for preventing the polymer latex, etc., from freezing and deteriorating even in cold climates was adopted.

Therefore, the problems are solved by the invention according to any one of the following 1 to 9.

Namely, the invention is characterized in that a teat pack material for lactating livestock is the material for forming a pack which adheres closely to the teat of lactating livestock, comprising at least (A) a polymer latex, and (B) a coagulant.

A particular embodiment is characterized in that the teat pack material for lactating livestock further comprises (C) a surfactant.

Another particular embodiment is characterized in that the teat pack material for lactating livestock further comprises (D) a release agent.

Another particular embodiment is characterized in that the teat pack material for lactating livestock further comprises (E) a water-soluble polymer.

Another further embodiment characterized in that the teat pack material for lactating livestock further comprises (F) water and/or a water-soluble solvent.

Another further embodiment is characterized in that the teat pack material for lactating livestock further comprises (G) an antifreezing agent.

Another further embodiment is characterized in that the (A) the polymer latex is an emulsion obtained by dispersing a natural or a synthetic rubber, or a synthetic polymer as the dispersoid in a dispersion medium mainly containing water.

Another further embodiment is characterized in that (B) the coagulant dissociates a divalent or more metal ion within the dispersion medium.

An additional embodiment is characterized in that (B) the coagulant is at least one type selected from citric acid, lactic acid, acetic acid, malic acid, fumaric acid, maleic acid, tartaric acid, gluconic acid, succinic acid, propionic acid, oxalic acid, benzoic acid or salts thereof.

A further embodiment is characterized in that (C) the surfactant is an anionic surfactant.

Another further embodiment characterized in that (D) the release agent is a compound comprising a -CF₃, -CF₂, -CH₃, or -CH₂ group.

A further embodiment is characterized in that (E) the water-soluble polymer is at least one type selected from methyl cellulose, carboxymethyl cellulose, hydroxyethyl cellulose, casein, polyvinyl alcohol, polyvinylpyrrolidone, polyvinyl methyl ether, polyacrylic acid, polymethacrylic acid, polyacrylamide, polyethylene oxide, and polyethyleneimide.

A further embodiment is characterized in that (G) the antifreezing agent is at least one type selected from the group consisting of ethylene glycol, propylene glycol, glycerin, alcohols, potassium acetate or calcium chloride.

Another aspect of the invention is a kit for forming the pack which adheres to the teat of lactating livestock, characterized in comprising (i) a base comprising at least (A) a polymer latex, stored in a predetermined container, and (ii) at least (B) a coagulant stored in a different container.

A further embodiment is characterized in that (i) the base or (ii) the coagulant invention further comprises (C) the surfactant.

Another further embodiment is characterized in that (i) the base or (ii) the coagulant further comprises (D) the release agent.

A further embodiment is characterized in that (i) the base or (ii) the coagulant further comprises (E) the water-soluble polymer.

An additional embodiment is characterized in that (i) the base or (ii) the coagulant further comprises (F) water and/or the water-soluble solvent.

Another further embodiment is characterized in that (i) the base or (ii) the coagulant further comprises (G) the antifreezing agent.

The present disclosure also proposes a method for preventing mastitis in lactating livestock characterized in that the teat pack for lactating livestock is formed by adhering one of (i) the base comprising at least (A) the polymer latex and (ii) at least (B) the coagulant to the teat of lactating livestock, and then adhering the other to the teat.

### Effects of the Invention

The teat pack material for lactating livestock of the present invention, the kit for forming the teat pack for lactating livestock, or the use of both for preventing mastitis in lactating livestock can obtain the effects exemplified below.
1. The teat pack material for lactating livestock of the present invention can easily and instantaneously prepare a teat pack having excellent uniformity (dispersability) of each component by anyone. Further, the pack is adhered to the teat, and will not peel off from the teat for a long period of time. Further, the material has a high elasticity, thus, the shape change due to the contraction and the expansion of the teat of the dairy cattle during the milking period can also be followed. The dairy cattle during the milking period can be effectively prevented from developing infectious diseases such as mastitis.
2. The teat pack material for lactating livestock of the present invention shows a good coatability on a teat coated with oil and fat, and the pack formed by the pack material has a good adhesiveness to the teat coated with oil and fat. Further, the strength of the pack is high, and thus, will not peel off from the teat for a long period of time. Therefore, the dairy cattle during the milking period can be effectively prevented from developing infectious diseases such as mastitis.
3. The teat pack material for lactating livestock of the present invention uses a polymer latex, thus, a pack in which the strength is high and having a high elasticity can be formed by a simple method, and even when the teats contact each other when lactating livestock lays down and rests, etc., the peeling of the pack which is thought to be caused by the adhesion of attached packs to each other can be prevented to a high degree. As a result, the dairy cattle during the milking period can be effectively prevented from developing infectious diseases such as mastitis.
4. The teat pack material for lactating livestock of the present invention is able to be quickly removed without using an additional facility, material, etc., when milking again or in case of emergency.
5. The teat pack material for lactating livestock of the present invention does not deteriorate even in cold climates.
6. The kit for forming the teat pack for lactating livestock of the present invention comprises (i) at least the base comprising (A) the polymer latex, stored in a predetermined container, and (ii) at least (B) the coagulant stored in a different container, and can achieve the effects exemplified below.
   (a) Careful handling is used in the transportation, storage, and preservation of latex, but the base comprising the polymer latex and the coagulant are respectively housed in individual containers, thus, long-term storage stability is guaranteed.
   (b) Merchandising is possible by appropriately changing the capacity of the respective containers in accordance with the scale of the dairy farmer.
   (c) The container which housed the base comprising the polymer latex, and the container which housed the coagulant respectively have a function as independent products, thus, it is possible to provide the necessary amount to necessary places when necessary in response to the requests of dairy farmers which leads to the expansion of applications.

### PREFERRED MODE FOR CARRYING OUT THE INVENTION

When using the teat pack material of the present invention, a pack which instantaneously adheres to ordinary teats and the teat coated with oil and fat of lactating livestock can be obtained by a simple means, and the pack will not peel off from the teat for a long period of time. The pack is produced by the coagulation of the polymer latex particles by adhering one of the base or the coagulant on the teat of lactating livestock, and then, adhering the other, and thus, can be obtained by a remarkably simple means. The pack is prepared by immersing the teat of lactating livestock in the teat pack material, and forming a film by natural drying for ten minutes or less without using forced drying such as heating.

Therefore, the vicinity of the teat canal is completely sealed, and even if the teat of the milking cattle repeatedly contracts and expands, the pack follows the shape change and can be maintained in a state adhered to the teat, thus, it is possible to reliably prevent the entry of mastitis pathogen into the teat canal.

Further, the teat pack material of the present invention does not require a kneading operation immediately before use, and thus, eliminates the problems such as the non-uniformity of the teat pack due to insufficient kneading and the entry of air bubbles.

The components constituting the teat pack material for protecting lactating livestock of the present invention will be explained in detail below.

### [(A) Polymer latex]

The polymer latex of the present invention is made by dispersing the particle consisting of the polymer in water or an organic solvent. "Latex" is originally the name given to the milky white aqueous emulsion collected from a natural rubber tree, and usually, latex refers to natural rubber latex (latex in the narrow sense). However, the colloidal sol which makes a rubber polymer such as synthetic rubber and synthetic resin as the dispersoid, and an aqueous solution of various organic solvents or inorganic matter as the dispersion medium is also referred to as latex (latex in the broad sense). The aqueous dispersion of synthetic rubber manufactured using an emulsion polymerization method is sometimes referred to as synthetic rubber latex to distinguish it from natural rubber latex. Likewise, the emulsion of a resin other than rubber is sometimes referred to as a resin polymer latex to distinguish it from rubber latex. However, recently, the term "polymer latex" has been used as the term which includes all of the natural rubber latexes, synthetic rubber latexes and resin latexes. Therefore, the present invention uses "polymer latex" below in the broad sense (refer to Soichi Muroi: The Chemistry of polymer latex, Kobunshi Kanko Kai (1976)). In the present invention, the solvent for dispersing the rubber polymer is preferably water from the viewpoints of the high stability and suppressing the cost.

The type of polymer latex of the present invention is not specifically limited, but, an aqueous dispersion of the rubber polymer is preferable in order to increase the strength of the pack. A small amount of organic solvent is permitted in the aqueous dispersion, and 10 mass% or less, and preferably 5 mass% or less of organic solvent may be present. The rubber polymer is preferably at least one type selected from, for example, natural rubber or a synthetic rubber such as isoprene rubber, butyl rubber, butadiene rubber, ethylene-butadiene copolymer rubber, ethylene-propylene rubber, styrene / butadiene copolymer rubber, styrene-isoprene copolymer rubber, urethane rubber, thiokol rubber, nitrile rubber, nitrile-butadiene copolymer rubber, chloroprene rubber, chlorosulfonated polyethylene rubber, chlorinated polyethylene rubber, acrylic rubber, vinyl acetate rubber, ethylene-vinyl acetate rubber, epichlorohydrin rubber, silicone rubber, polysulfide rubber, polyether rubber, fluororubber, polysulfide rubber, or an aqueous dispersion of a derivative thereof. Note that, the derivatives of the rubber polymer refer to, as shown below, the compounds obtained by copolymerizing rubber polymers with each other, and the compounds which denatured the surface of the rubber polymer to a reactive functional group such as a carboxy group. Among the rubber polymers mentioned above, rubber polymers whose surface has a negative charge is preferred, because when an electrolyte or acid (hydrogen ion) is added, the charge of the rubber polymer surface is neutralized and the repulsive force between rubber polymers disappears, so that the rubber polymers adhere to each other more rapidly and coagulate. Further, among the aforementioned rubber polymers, an aqueous dispersion of the rubber polymer having a large elongation and a high strength in order to withstand the expansion and the contraction of the teat of lactating livestock is more preferable. The polymer latex manufactured from an aqueous dispersion of a natural rubber or isoprene rubber is preferable from the viewpoints of the ease of coagulation and having a large elongation and a high strength. Among the natural rubbers or isoprene rubbers, those in which a part was crosslinked beforehand have a higher strength and are preferred. The aforementioned polymer latex may be a mixture of two or more thereof, and may be copolymerized with each other. The form of the copolymerization may be any of random copolymerization, alternating copolymerization, periodic copolymerization, block copolymerization, graft copolymerization. The structure of the polymer within the polymer latex particles may be any of linear, branched, dendrimeric, networked, cyclic, etc. Further, a compound which denatures the surface of the polymer latex particles to a reactive functional group such as a carboxy group may be used. Compounds in which various stabilizers such as ammonia and surfactants such as glycerol fatty acid esters and laurate ester and a surfactant were added may be used in order to stabilize the polymer latex particles. Further, in accordance with need, a vulcanizing agent, a vulcanization accelerator, a vulcanization acceleration aid, a vulcanization retardant, a deterioration inhibitor (antioxidant, ozone inhibitor, etc.), processing aids (plasticizers, softeners, tackifiers, etc.), dispersants, creaming agents, foaming agents, heat-sensitive agents (zinc ammonium complex salts, polyvinyl methyl ether, etc.) may be added. The solid component concentration within the polymer latex is preferably 20 mass% to 90 mass%, and more preferably 40 mass% to 85 mass%. When the solid component concentration is less than 20 mass%, it requires time to form the pack, and the strength of the pack tends to decrease. However, when the solid component concentration is more than 90 mass%, the viscosity of the base increases, and the coatability to the teat of the pack tends to decrease.

Furthermore, at present, a wide variety of synthetic resin-based polymer latexes such as vinyl chloride type, acrylic type, vinyl acetate type, acrylic ester copolymer type, vinyl acetate copolymer type, vinyl acetate-acrylic type, ethylene-vinyl acetate type, vinyl chloride-acrylic type, styrene-acrylic type, silicone-acrylic type, silicone-urethane type, silicone-acrylic type, fluorinated acrylic type, etc., having a versatile solid content, viscosity, pH, an average particle size, a glass transition point, a minimum film-forming temperature, an acid value, etc., have been marketed. Thereamong, a polymer latex which is suitable in the present invention can be selected. For example, when it is desired to impart releasability, a silicone-based or a fluorine-based polymer latex can be selected.

### [(B) Coagulant]

(B) The coagulant in the present invention is selected from the group consisting of, for example, low molecular inorganic coagulants, polymer inorganic coagulants, polymer organic coagulants, acids, and mixtures thereof.
For example, the hydrophilic groups (-SO₃H, -SO₃M, -OSO₃H, -OSO₃M, -COOH, -NR₃X, - COOH, -NH₂, -CN, -OH, -NHCONH₂, -(OCH₂CH₂)ₙ-, -CH₂OCH₃, -OCH₃, COOCH₃, -CS, etc., [R is alkyl group, M is alkali metal or NH₄, and X is halogen]) of the emulsifier molecules are bonded to the surface of the polymer particulate constituting the polymer latex to have a negative charge. The coagulant has a role in neutralizing the charge of the polymer particulate surface constituting the polymer latex, and coagulating the particles.

### 1. Low molecular inorganic coagulant (metal salt)

One (B) coagulant of the present invention is a low molecular inorganic coagulant. The low molecular inorganic coagulant is exemplified by a wide variety of metal salts. Among the coagulants, specifically, the metal salt effectively causes the coagulation of the latex particles having such a negative charge. The metal salt functioning as the coagulant in the present invention is defined in a broad sense as the compound in which an anion derived from an acid and a cation derived from a base are ionically bonded, and is a compound generated by a neutralization reaction between an acid and a base, a reaction between an acid and a basic oxide, a reaction between an acid and a metal simple substance, a reaction between a base and an acidic oxide, a reaction between a base and a nonmetal simple substance, a reaction between an acidic oxide and a basic oxide, and a reaction between a nonmetal simple substance and a metal, and is capable of being ionized within the dispersion medium of the latex to produce metal ions. The metal salt functioning as the coagulant in the present invention is not specifically limited to the metal salt having the aforementioned function, and all of the well-known metal salts can be used, for example, sodium chloride, potassium chloride, calcium chloride, magnesium chloride, iron chloride, tin chloride, aluminum chloride, titanium chloride, sodium sulfate, potassium sulfate, calcium sulfate, magnesium sulfate, zinc sulfate, aluminum sulfate, iron (II) sulfate, iron (II) sulfate, iron (III) chloride, zirconium sulfate, zinc oxide, magnesium oxide, aluminum oxide, iron oxide, titanium oxide, zirconium oxide, tin oxide, sodium hydroxide, potassium hydroxide, zirconium hydroxide, tin hydroxide, calcium acetate, zinc acetate, sodium lactate, potassium lactate, calcium lactate, zinc lactate, potassium fluorotitanate, ferruginous aluminum sulfate, ammonium alum, potassium alum, iron chloride-iron sulfate mixture, etc., can be exemplified. These may be in a mixture of two or more.

Regarding the ease of coagulation of the polymer particles from the polymer latex due to the salting out action by the metal salt, generally, the greater the valence number of the ion having a charge opposite to the charge of the particle which is the dispersoid, the more remarkably the particle charge is neutralized, thus, the polymer latex is easily coagulated. For example, the coagulation force order of the polymer latex in the case of a monovalent cation is Li > Na > K > Rb > Cs, and similarly, the coagulation force order in the case of a divalent cation is Mg > Ca > Sr > Ba. Naturally, considering the toxicity to lactating livestock and operators which are living organisms, and the reactivity with water, magnesium salt which dissociates into Mg²⁺ ion or calcium salt which dissociates into Ca²⁺ ion is preferable.

### 2. Polymer inorganic coagulant

Aluminum polychloride ([Al₂(OH)nCl₆₋ₙ]ₘ), aluminum polysulfate ([Al₂(OH)ₙ(SO₄)_{3-n/2}]ₘ), iron (III) polychloride ([Fe₂(OH)ₙCl₆₋ₙ]ₘ), iron (III) polysulfate ([Fe₂(OH)ₙ(SO₄)_{3-n/2}]ₘ) may be exemplified as the polymer inorganic coagulant.

### 3. Polymer organic coagulant

As the polymer organic coagulants used as the coagulant in the present invention, polyacrylamide, polyethylene oxide, urea-formalin resin, etc., are exemplified as the [nonionic] coagulant, sodium polyacrylate (acrylamide-sodium acrylate copolymer), a polyacrylamide partial hydrolysate, sulfomethylated polyacrylamide, polyaminoalkyl(meth)acrylate, halogenated polyvinyl pyridium, halogenated polydiallyl ammonium, etc., are exemplified as the [anionic] coagulant, and poly-amino methyl acrylamide, polyvinyl imidazoline, chitosan, epoxy-amine, etc., are exemplified as the [cationic] coagulant. These coagulants have molecular weights from several tens of thousands to several millions, and the greater the molecular weight, the greater the charge becomes.

### 4. Acids

The type of acid used as the coagulant for coagulating the polymer particulate constituting the polymer latex is not specifically limited, but citric acid, lactic acid, acetic acid, malic acid, fumaric acid, maleic acid, tartaric acid, gluconic acid, succinic acid, propionic acid, butyric acid, sulfuric acid, etc., may be exemplified. These acids may be the metal salts thereof. The order of the ability to coagulate the ions into which these acids dissociate, the so-called coagulation force order is citric acid ion > tartaric acid ion > sulfuric acid ion > acetic acid ion > Cl⁻ > Br⁻ > NO₃⁻ > ClO₃⁻ > I⁻ > SCN⁻, etc. When selecting an acid or a salt thereof as the coagulant for coagulating the polymer latex in the present invention, the safety to living organisms such as lactating livestock and the operators, user-friendliness, etc., should be taken into consideration with reference to the coagulation force order. Note that, these acids or the salts thereof may be used singly or may be a mixture of two or more, and, one or two or more of the above coagulants may be used together.

### [(C) Surfactant]

One preferred embodiment of the present invention lies in blending the surfactant in the teat pack material comprising the latex. By blending the surfactant, not only are the latex particles stabilized within the dispersion medium, but there is also the effect of improving the affinity to the teat coated with oil and fat, and improving the coatability of the pack material to the teat.

Well-known surfactants can be utilized without any specific limitation, and any of anionic surfactants, cationic surfactants, amphoteric surfactants and nonionic surfactants can be used. An anionic surfactant is preferably used in order to further improve the coatability of the pack composition on a teat which is greasy. These surfactants may be used singly or a combination of two or more may be used.

For example, alkyl carboxylate, alkyl sulfate ester salt, alkylsulfonic acid salt, alkylbenzene sulfonate, alkyl ether carboxylate, etc., may be provided as the anionic surfactant. Thereamong, alkyl carboxylate, alkyl sulfate ester salt, and alkylsulfonic acid salt having a C6 to C18 alkyl group are preferable, and ammonium laurate, sodium lauryl sulfate, sodium dodecanesulfonate, etc., are preferably used.

For example, alkylamine salt and quaternary ammonium salt, etc., may be provided as the cationic surfactant. Thereamong, quaternary ammonium salt is preferable, and for example, tetrapropylammonium chloride may be provided.

For example, aminocarboxylate, etc., may be provided as the amphoteric surfactant. Polyoxyethylene alkyl ether, polyoxyethylene alkylphenyl ether, polyoxyethylene-polyoxypropylene block polymer, glycerol fatty acid esters, polyglycerol fatty acid esters, polyoxyethyleneglycerol fatty acid esters, polyoxyglycerol fatty acid esters, sorbitan fatty acid ester, sucrose ester, polyoxydiethylene alkylamine, block polymers of polyoxysiloxanes and polyoxyethylenes, etc., may be provided as the nonionic surfactant.

Polyglycerol fatty acid ester, etc., may be provided as the nonionic surfactant. The compound obtained by the esterification reaction with 1:1 to 1:4 of polyglycerin and fatty acid by a conventional method, and the main component is the part of the polyglycerin having the hydroxy group as the compound which is ester bonded with the fatty acid may be provided as the polyglycerol fatty acid ester. Monoglycerin, a polyglycerin having a degree of polymerization of n = 1 to 10 such as diglycerin, triglycerin, tetraglycerin, and decaglycerin can be used as the polyglycerin. A C8 to C18 fatty acid such as lauric acid, myristic acid, stearic acid, and oleic acid may be provided as a fatty acid which forms an ester, and these may be used singly or a mixture of two or more types can be used. For example, decaglyceryl trioleate may be provided as such a polyglycerol fatty acid ester. An anionic surfactant is preferably used in order to further improve the coatability of the teat pack material for lactating livestock onto the oil and fat coated teat. The probable reason for this is that there are many cases when the latex particle surface is anionically (negative) charged, and in this case, if using a cationic surfactant, it is considered that there is the case when the deterioration of the coatability which causes the insufficient dispersion of the latex occurs due to the electrostatic interaction. Conversely, in the case of an anionic surfactant, it is considered that such a situation is unlikely to occur. Note that, the surfactant may be used singly or a combination of two or more may be used.

The content of the surfactant is not specifically limited, but, it is preferable to adjust the contents as follows from the viewpoints of the coatability to the teat coated with oil and fat and the pack strength generated.

For the teat pack material to be stored without separating into the base and the coagulant, the content of the surfactant relative to the entirety of the teat pack material for lactating livestock is preferably made from 0.05 mass% to 10 mass%, and more preferably from 0.10 mass% to 5 mass%. When the content of the surfactant is less than 0.05 mass%, the coatability to the teat coated with oil and fat tends to become somewhat poor. However, when the content of the surfactant is above 10 mass%, the strength of the formed pack tends to decrease slightly.

For the teat pack material to be stored by separating into the base and the coagulant, the surfactant may be added to the base or the coagulant, but the coatability of the teat pack material to the teat coated with oil and fat further increases, thus, it is preferable to add to the base. When adding the surfactant to the base, the content of the surfactant relative to the entirety of the base is preferably from 0.05 mass% to 10 mass%, and more preferably from 0.10 mass% to 5 mass%. When the content of the surfactant is less than 0.05 mass%, the coatability to the teat coated with oil and fat tends to become somewhat poor. On the other hand, when the content of the surfactant is above 10 mass%, the strength of the formed pack tends to decrease slightly. When adding the surfactant to the coagulant, the content of the surfactant relative to the entirety of the coagulant is preferably from 0.05 mass% to 10 mass%.

### [(D) Release agent]

The teat pack material of the present invention, as a preferred embodiment, is characterized in comprising (D) a release agent. By comprising (D) the release agent, not only is it possible to prevent the adhesion of the packs to each other after using the teat pack material and producing the packs, but also the pack can be quickly removed without using additional facility, material, etc., when milking again or in case of emergency.

The type and the blending amount of (D) the release agent may be appropriately selected so that the teat pack material for lactating livestock of the present invention has an adhesive force, i.e., a force required for peeling, in the 180° direction, two films which are brought into contact with each other with a pressure of 1 N of 0.3 N/10 mm or less, provided that the two films are formed of the teat pack material for lactating livestock of the present invention in a thickness of 0.1 mm on a PET film having a width of 20 mm, a length of 100 mm, and a thickness of 0.1 mm.

The release agent used in the present invention is defined in the broad sense as a compound having a -CF₃, -CF₂, -CH₃, or CH₂ group, and in the narrow sense, is defined as a compound having a saturated hydrocarbon moiety such as, a methane-based hydrocarbon, a paraffin-based hydrocarbon, or and alkane, or a compound having a number of methyl groups such as a silicone resin, or a fluorocompound having a perfluoroalkyl group. Note that, the ability (effect) as a release agent is in the order of the compounds comprising a -CF₃ > -CF₂ > - CH₃ > -CH₂ group. As a specific example, a compound (silicon-based release agent) comprising silicon atoms such as organopolysiloxane and silicone oil, a compound (fluorine-based release agent) comprising fluorine atoms such as polytetrafluoro ethylene and perfluoroalkyl group containing polymer, and additionally, well-known release agents such as polyethylene wax and amide wax may be used. From the viewpoints of the miscibility with (A) the polymer latex and the safety to humans and lactating livestock, a compound containing a silicon atom or a fluorine atom is preferable, and silicone oil which is a compound comprising silicon atoms is preferably used. Specifically, the characteristics of the silicone oil are that it (a) has a high flash point and is a flame retardant, (b) has a high resistance to shear and a large compressibility, (c) shows a distinctive lubricity, (d) is chemically inert, and (e) is physiologically substantially harmless, and thus, silicone oil has been used in cosmetics, medical, and food use, and is preferable from the viewpoints of safety and the physical properties, from the viewpoint of food sanitation, and from the viewpoint of safety in the workplace.

Any of a straight silicone oil such as dimethyl silicone oil, methylphenyl silicone oil, methyl hydrogen silicone oil, or modified silicone oil may be used as the silicone oil. Amino-modified silicone, alcohol-modified silicone, vinyl-modified silicone, urethane-modified silicone, polyester-modified silicone, polyether-modified silicone, polyester-modified silicone acrylic-modified silicone, aralkyl-modified silicone, and amide-modified silicone may be provided as the modified silicone oil. Specifically, when the dispersion medium of the polymer latex particles is water, a polyether-modified silicone oil having a good compatibility with water is preferably used.

The content of (D) the release agent may be appropriately selected so that the teat pack material for lactating live stock of the present invention has an adhesive force, i.e., a force required for peeling, in the 180° direction, two films which are brought into contact with each other with a pressure of 1N of 0.3 N/10 mm or less, provided that the two films are formed of the teat pack material for lactating livestock of the present invention in a thickness of 0.1 mm on a PET film having a width of 20 mm, a length of 100 mm, and a thickness of 0.1 mm, but in order that a sufficient releasability is provided to the pack while sufficiently maintaining the film strength of the pack, thus, for the teat pack material to be stored without separating into the base and the coagulant, the concentration within the entirety of the teat pack material is preferably from 0.5 mass% to 10 mass%. For the teat pack material to be stored by separating into the base and the coagulant, when the release agent is added to the base, the content of the release agent relative to the entirety of the base is preferably from 0.5 mass% to 10 mass%. On the other hand, when the release agent is added to the coagulant, the content of the release agent relative to the entirety of the coagulant is preferably 0.5 mass% or more 10 mass% or less. From the viewpoint of strongly preventing the adhesion, the adhesive force is preferably made to 0.2 N/10 mm or less. By adjusting the adhesive force in the range specified by the present invention, the adhesion of the packs to each other can be prevented, and it becomes possible to remove the packs after use.

The teat pack material for lactating livestock of the present invention has preferably has an adhesive force, i.e., a force required for peeling two films which are brought into contact with each other with a pressure of 1 N in the 180° direction, of 0.3 N/10 mm or less, provided that the two films are formed of the teat pack material for lactating livestock of the present invention in a thickness of 0.1 mm on a PET film having a width of 20 mm, a length of 100 mm, and a thickness of 0.1 mm. The adhesive force of the present invention is measured as follows.

The entire surfaces of the two PET films (a width of 20 mm, a length of 100 mm, and a thickness of 0.1 mm) were coated so that the teat pack material of the present invention became a thickness of 0.1 mm. After 30 minutes had elapsed after coating, the film (cured teat pack material) laminated on the two PET films was stuck together in a condition in which a pressure of 1N was applied so as to be brought in contact. Then, the film was stripped off by a tensile testing machine under the conditions of stripping in the 180° direction at a speed of 200 mm/min, and the value obtained by dividing the peeling force (the load (N) required for stripping) at this time by 2 was defined as the adhesive force (N/10 mm). Note that, the measurement of the adhesive force was performed at 23°C.

### [(E) Water-soluble polymer]

In a preferred embodiment of the present invention, (E) the water-soluble polymer is blended in the teat pack material. Similar to the surfactant, the water-soluble polymer is adsorbed in the interface of the polymer microparticles constituting the latex to form the protective layer. Examples of the water-soluble polymer used in the present invention are at least one type selected from methyl cellulose, carboxymethyl cellulose, hydroxyethyl cellulose, casein, polyvinyl alcohol, polyvinylpyrrolidone, polyvinyl methyl ether, polyacrylic acid, polymethacrylic acid, polyacrylamide, polyethylene oxide and polyethyleneimide. Polymer latexes in which the respective viscosities are different are commercially available, but the viscosity of a polymer latex cannot be changed to a wide extent, thus, in the present invention, the water-soluble polymer is preferably used as a thickening agent in accordance with need to prepare the final viscosity of the polymer latex.

### [(F) Water and/or the water-soluble solvent]

In the present invention, (B) the coagulant selected from the group consisting of a low molecular inorganic coagulant, a polymer inorganic coagulant, a polymer organic coagulant, an acid, and mixtures thereof are used as the coagulant which coagulates the polymer particulates as the dispersoid constituting the polymer latex. When a commercially available polymer latex is used as the base, there are cases when (B) the coagulant is not completely dissolved in the amount of water blended as the dispersion medium. Specifically, this tends to occur when a metal salt, a polymer inorganic coagulant, a polymer organic coagulant, or a solid acid is used as the coagulant. In this case, it is preferable to further add (F) water and/or the water-soluble solvent to the coagulant side. The type of water-soluble solvent is not specifically limited so long as (B) the coagulant is completely dissolved and is not harmful to the human body or lactating livestock, and all of the well-known solvents can be used. Thereamong, ethanol, propanol, butanol, acetone, methylethylketone, methanol, isopropyl alcohol, etc., are preferable. The blending amount of water and/or the water-soluble solvent is not specifically limited, but the addition amount of water and/or the water-soluble solvent relative to 100 parts by mass of (B) the coagulant is preferably 150 parts by mass to 4000 parts by mass, and more preferably 200 parts by mass to 3000 parts by mass. When the addition amount of water and/or the water-soluble solvent relative to 100 parts by mass of (B) the coagulant exceeds 4000 parts by mass, there is the possibility that the coagulation of the latex will not occur instantaneously. On the other hand, when the blending amount of water and/or the water-soluble solvent relative to 100 parts by mass of (B) the coagulant does not reach 150 parts by mass, there are cases when (B) the coagulant does not sufficiently dissolve in water and/or the water-soluble solvent. As stated above, the blending of the water and/or the water-soluble solvent was explained from the function of completely dissolving (B) the coagulant. However, a polar solvent may function as a coagulant for coagulating the polymer particulate as the dispersoid constituting the polymer latex. Insofar as this function is concerned, when the charge of the polymer particulate is anionic, if the water-soluble solvent is added in the range of about 0.5 to 40% relative to the weight of the latex, the polymer particles coagulate from the latex and a synergistic effect with (B) the coagulant can be anticipated.

### [(G) Antifreezing agent]

Commercially available polymer latexes usually undergo a freezing-thawing cycle during the storage and the preservation. When freezing, in mild cases, the viscosity remains within the upper range, but in extreme cases, problems such as the polymer latex being completely frozen so that it cannot be used when use is desired occur. In a preferable embodiment of the present invention, the problem was solved by blending the antifreezing agent in the teat pack material in advance. The antifreezing agent used in the present invention is at least one type selected from ethylene glycol, propylene glycol, glycerin and alcohols. Potassium acetate or calcium chloride may be added therein. By blending these antifreezing agents, the teat pack material of the present invention does not freeze until about -60°C or less. The content of the antifreezing agent in the present invention is preferably at least about 9 mass% of the polymer latex, and if 3 to 9 mass%, the entirety of the polymer latex completely solidifies, or partially coagulates depending on the type of polymer which is the dispersoid and the surfactant and the particle size of the polymer particle.

### [Additives]

In addition to each component explained above as main components, each type of additive can be blended in the pack material for lactating livestock of the present invention in accordance with need. As additives, in addition to, for example, sterilizing disinfectants, fillers, thickening agents, coloring agents, fragrances, preservatives, etc., various additives (materials) which are commonly used in the processing of plastics or rubbers such as plasticizers, stabilizers, cross-linking agents (vulcanizing agent and a vulcanization accelerator), and petroleum-based softening agents for rubber can be appropriately selected and blended.

### [Storage form, etc.]

Regarding the teat pack material for lactating livestock of the present invention, it is preferable that the base comprising (A) the polymer latex and (B) the coagulant selected from the group consisting of the low molecular inorganic coagulant, the polymer inorganic coagulant, the polymer organic coagulant, the acid, and mixtures thereof are separately stored. The most ideal storage form is to house (i) the base comprising at least (A) the polymer latex in a predetermined container, and to house (ii) at least (B) the coagulant selected from the group consisting of the low molecular inorganic coagulant, the polymer inorganic coagulant, the polymer organic coagulant, the acid, and mixtures thereof in a different container, and a kit is produced by combining. The effect, and the like due to the kit was already explained, thus, an explanation thereof is omitted.

Further, in order to store as the kit, (F) water and/or the water-soluble solvent is added to a predetermined container, and then, (B) the coagulant selected from the group consisting of the low molecular inorganic coagulant, the polymer inorganic coagulant, the polymer organic coagulant, the acid, and mixtures thereof is preferably added to dissolve the coagulant and make the coagulant solution uniform. By using a uniform coagulant solution liquid, the uniformity of the configuration of the pack becomes good, and the adhesiveness can be further improved. The blending amount of (F) water and/or the water-soluble solvent is not specifically limited, but, the blending amount of (F) the water and/or the water-soluble solvent relative to 100 parts by mass of (B) the coagulant selected from the group consisting of the low molecular inorganic coagulant, the polymer inorganic coagulant, the polymer organic coagulant, the acid, and mixtures thereof is preferably 150 parts by mass to 4000 parts by mass, and more preferably 200 parts by mass to 3000 parts by mass. When the blending amount of (F) the water and/or the water-soluble solvent is in excess of 4000 parts by mass relative to 100 parts by mass of (B) the coagulant selected from the group consisting of the low molecular inorganic coagulant, the polymer inorganic coagulant, the polymer organic coagulant, the acid, and mixtures thereof, there is the possibility that the coagulation of the polymer latex will not occur instantaneously. However, when the blending amount of (F) the water and/or the water-soluble solvent does not reach 150 parts by mass relative to 100 parts by mass of (B) the coagulant selected from the group consisting of the low molecular inorganic coagulant, the polymer inorganic coagulant, the polymer organic coagulant, the acid, and mixtures thereof, there are cases when (B) the low molecular inorganic coagulant, the polymer inorganic coagulant, or the polymer organic coagulant does not sufficiently dissolve in (F) water and/or the water-soluble solvent.

### [Sterilizing disinfectant]

A sterilizing disinfectant may be used in order to impart a sterilizing and disinfecting effect to the pack material and prevent an increase in the number of pathogen in the vicinity of the teat canal. The sterilizing disinfectant is not specifically limited as long as the sterilizing disinfectant can kill a wide range of harmful microbes such as various bacteria, fungi, and viruses which cause diseases such as mastitis, and the sterilizing disinfectant can be exemplified by, for example, an iodine compound, metals such as silver, copper, zinc, titanium, and iron and metal salts, tea leaf powder, hinoki powder, chitosan, benzalkonium chloride, benzethonium chloride, fatty acid ester such as caprylic acid monoglyceride, triclosan, isopropylmethylphenol, cetylpyridinium chloride, resorcin, trichlorocarbanide, halocarbon, chlorhexidine, chlorhexidine hydrochloide, chlorhexidine gluconate, acrinol, sodium hypochlorite, hydrogen peroxide, etc. The sterilizing disinfectant may be added to both the base and the coagulant or may be added to one thereof.

Thereamong, iodine compounds and silver are suitable from the viewpoints of skin irritation to humans or lactating livestock, sustainability of the sterilizing and disinfecting effect, and cost. For example, iodine, povidone iodine, sodium iodate, potassium iodate, sodium iodide, potassium iodide, iodoform, etc., may be provided as the iodine compound.

The blending amount of the sterilizing disinfectant is not specifically limited, but, is usually preferably contained in a range of 0.1 to 10 mass% within the base or the coagulant.

### [Filler]

A filler may be used to adjust the physical properties of the pack, mainly the reinforcing effect, the thickening effect, etc. The filler may be added to both the base and the coagulation liquid or may be added to one thereof. Oxides of metals or semimetals such as silica and alumina, inorganic fluorine compounds such as potassium fluorotitanate and potassium fluorosilicate, particles of silicone resin, etc., are preferably used as the filler. The blending amount of the filler is not specifically limited, but, is usually preferably contained in a range of 0.5 to 30 mass% within the base or the coagulant.

### [Thickening agent]

Further, a thickening agent may be used in order to adjust the viscosity of the base and/or the coagulant (liquid), and to improve the yield of the base and/or the coagulant (liquid) to the teat. The thickening agent may use one of or a combination of the inorganic thickening agents shown below and a synthetic petroleum-based thickening agent.

The inorganic thickening agent is generally a compound such as colloidal magnesium aluminum silicate and a colloidal clay, and these are fumed or precipitated to make particles having a large surface-size ratio.

Water-soluble polymers such as polyvinyl alcohol, vinylpyrrolidone, methyl vinyl ether, polyacrylic acid, polymethacrylic acid, polyacrylamide, polyethylene oxide, methyl cellulose, hydroxyethyl cellulose, casein, or ethyleneimine may be generally exemplified as thickening agents for polymer latex.

### [Coloring agents]

Coloring agents may be used in order to color the pack and increase the visibility of the pack. The type of coloring agent is not specifically limited, but, titanium oxide, zinc oxide, barium sulfate, calcium carbonate, etc., may be provided as a preferred example of a white pigment. Further, if using coloring agents which have a complementary color relation with the color of the teat, it becomes easier to see when a crack occurs in the pack. As examples of these coloring agents, carbon black, aniline black, etc., are exemplified as black pigments, iron oxide yellow is exemplified as a yellow dye or pigment, and Bengal red, alizarin red, etc. are exemplified as the red dye or pigment. When using coloring agents, a contraction difference (the contraction ratio = the contraction rate in the flow direction/the contraction rate in the perpendicular direction) is generated due to the direction in which the molecule is oriented depending on the addition amount (%) of the coloring agents and causes twisting and distortion of the molded product, thus, the addition amount (%) of the coloring agents must be determined taking this point into consideration. From this viewpoint, titanium oxide (white pigment), cadmium yellow (yellow), carbon black (black pigment), etc., are preferable, as the contraction ratio (= the contraction rate in the flow direction/the contraction rate in the perpendicular direction) is almost unchanged in the case when there is no addition of the coloring agents. From the viewpoint of the contraction ratio, the addition amount of the coloring agents is preferably about 0.5 mass% or less.

### [Production method and usage method]

The present invention uses the compounds selected from the group consisting of (a) the low molecular inorganic coagulant, (b) the polymer inorganic coagulant, (c) the polymer organic coagulant, (d) the acid, and (e) mixtures thereof as the coagulant which coagulates (A) the polymer latex. Therefore, there are cases when the five types of compounds of (a) the low molecular inorganic coagulant, (b) the polymer inorganic coagulant, (c) the polymer organic coagulant, (d) the acid, and (e) mixtures thereof are collectively referred to as "(B) the coagulant" in explanations such as of the following production method and usage method.

### 1.[Production method -1 of (A) the base and (B) the coagulant]

The production method of the coagulant liquid when dissolving mainly (A) the base comprising the polymer latex, (B) the coagulant, etc., in (F) water and/or the water-soluble solvent is not specifically limited, but can be prepared using a well-known agitation mixer. In this case, examples of the agitation mixer which may be utilized include a rotating container type mixing kneader such as a ball mill, and a fixed container type mixing kneader having a horizontal axis or a vertical axis such as a ribbon mixer, a Ko-kneader, an internal mixer, a screw kneader, a Henschel mixer, a versatile mixer, a Loedige mixer, or a butterfly mixer. Note that, in the production of the coagulation liquid agent, when (B) the coagulant, etc., has a relatively high solubility in (F) water and/or the water-soluble solvent, an agitation apparatus in which no strong shearing force is applied to the component to be dissolved or a solution having the component dissolved therein may be utilized. As such an agitation apparatus, for example, a transferable agitator, a vertical agitator, and a side entering agitator, equipped with various impellers, and a line agitator may be used. Furthermore, in the preparation of the aforementioned base and the coagulation liquid (agent), two or more kinds of the various mixing kneaders may be used in combination.

### 2.[Method for formation of the pack]

The teat pack for lactating livestock (hereinafter, simply referred to as the "pack") of the present invention is formed by, for example, preparing (i) at least (A) the base comprising the polymer latex, and (ii) at least (B) the coagulant, adhering one thereof to the teat, and then adhering the other thereof. After adhering the base to the teat, it is preferable to immediately adhere the coagulant to the teat.

The immersion method, the brush coating method, a spraying method, etc., may be provided as the method (adhering method) for coating the teat with the base and the coagulant, but the adhering method is not specifically limited thereto. The immersion method is preferable.

The viscosity of the base and the coagulant in the case when using the immersion method may be appropriately selected so that the teat is easily immersed in each solution.

The viscosity of the base and the coagulant when using the immersion method is the value measured by a cone-and-plate viscometer at 23°C, and is preferably in the range of 1 to 300 dPa·s.

Note that, the viscosity of the base and the coagulant when using the spray method is preferably in the range of 1 to 100 dPa·s from the viewpoint of the ease of spraying.

When using the immersion method, the teat pack material (for the teat pack material to be stored by separating into the base and the coagulant, each of the base and the coagulant) is placed in a cylindrical or cup-shaped container which can accommodate, for example, the teat of lactating livestock. Next, after the container is moved in the direction of the base of the teat (pulling up), and the teat is immersed in the solution within the container, the operation for moving the container in the direction of the tip of the teat (pulling down) is performed (for the teat pack material to be stored by separating into the base and the coagulant, the operation is performed on each of the base and the coagulant). The immersion may be performed at a length criteria which makes the teat length to 100% so that 10% or more and preferably 20% or more of the teat is immersed in the teat pack material (for the teat pack material to be stored by separating into the base and the coagulant, each of the base and the coagulant). The immersion degree (immersion depth) of the teat into the teat pack material (for the teat pack material to be stored by separating into the base and the coagulant, the operation is performed on each of the base and the coagulant) is not specifically limited, but, is preferably performed at an immersion depth so that the cow does not feel an uncomfortable feeling in the teat. The immersion time of the teat is preferably two seconds or more in order to sufficiently and respectively adhere the base and the coagulant to the teat.

The material of the container is not limited so long as it is clean, and any of metal, ceramics, plastics, paper and the like can be used. Further, the container may have the minimum internal volume necessary to adhere the base or the coagulant to the teat in order to reduce the waste of the base and the coagulation liquid. In the case of a cylindrical or cup-shaped container, a container having an inner diameter from about 4 cm to about 6 cm, and a height of about 2 cm to about 10 cm can be used. It is convenient to have an index in order to be able to place a suitable amount of the base or the coagulant on the inside of the container.

The speed (pulling down speed) for moving the container in the direction of the tip of the teat after the immersion is preferably set to 5 to 100 mm/second, and is more preferably 10 to 50 mm/second in order to uniformly adhere the base and the coagulant to the teat.

The base and the coagulant are preferably adhered to the teat without an interval therebetween in order that the coagulant is adhered to the teat to form the pack in a state in which the base or the coagulant are sufficiently adhered to the teat.

The ratio (base / coagulant [parts by mass / parts by mass]) of the adherence amount of the base and the coagulant to the teat is not specifically limited, but normally is preferably in the range of 0.5 to 2.

### 3.[Teat pack for lactating livestock]

The teat pack for lactating livestock of the present invention is directly formed on the teat of lactating livestock, as explained in, for example, the aforementioned 2.[Method for formation of the pack]. The pack for lactating livestock can be used in the protection of the teat of lactating livestock. Specifically, the pack for lactating livestock can be used for the protection from infectious mastitis, the protection from environmental mastitis, and the protection from contamination and other external environmental factors. In the present invention, lactating livestock are specifically, lactating livestock for milking, and, for example, are dairy cattle, goats, and other lactating livestock in which milking is performed.

These points were already explained, thus, an explanation thereof is omitted.

### 4.[Production method -2 of (A) the base and (B) the coagulant]

The teat pack material for lactating livestock of the present invention preferably comprises (C) a surfactant in addition to (A) the base comprising the polymer latex and (B) the coagulant. The production method when the pack material for lactating livestock comprises (A) the base comprising the polymer latex, (B) the coagulant, and (C) the surfactant will be explained below.

The teat pack material for lactating livestock comprising (C) the surfactant can be adjusted by mixing (A) the polymer latex, and (C) the surfactant, (B) the coagulant and furthermore, each type of additive in accordance with need. For the teat pack material to be stored by separating into the base and the coagulant, the base and the coagulant can be mixed and prepared in the same manner as each of the constituent components of the base and the coagulant. The production method was explained in 1.[Production method -1 of (A) the base and (B) the coagulant], thus, an explanation thereof is omitted.

The type and the blending amount of (C) the surfactant, the storage form of (A) the base and (B) the coagulant of the teat pack material, etc., were already explained, thus, an explanation thereof is omitted.

### 5.[Method for formation of the pack]

The pack of the present invention is produced by adhering the teat to the teat pack material comprising at least (A) the base comprising the polymer latex, and (C) the surfactant, etc. For the teat pack material to be stored by separating into the base and the coagulant, after adhering one thereof to the teat, the other is adhered to the teat to form the pack, but in order to form uniform pack even to a greasy teat, the coagulant is preferably adhered to the teat after adhering the base to the teat.

In addition, the specifics relating to the coating method, the container, the teat pack for livestock, etc., are the same as the contents explained in 1.[Production method -1 of (A) the base and (B) the coagulant], thus, an explanation thereof is omitted.

### 6.[Production method -3 of (A) the base and (B) the coagulant]

The teat pack material for lactating livestock of the present invention preferably comprises (D) a release agent in addition to (A) the base comprising the polymer latex and (B) the coagulant. The production method when the pack material for lactating livestock comprises (A) the base comprising the polymer latex, (B) the coagulant, and (D) the release agent will be explained below.

### [Production method of the teat pack material]

The production method of the teat pack material is not specifically limited, but (A) the latex, (B) the coagulant, (D) the release agent, and (F) water and/or the water-soluble solvent, the additives, etc., can be mixed and prepared using a well-known agitation mixer in accordance with need. For the teat pack to be stored by separating into (A) the base and (B) the coagulant, the compounds can be mixed and prepared using a well-known agitation mixer in the same manner as each constituent component of the base and the coagulant.

The production method was explained in 1.[Production method -1 of (A) the base and (B) the coagulant], thus, an explanation thereof is omitted.

The type and the blending amount of (D) the release agent, and the storage form and the like of (A) the material and (B) the coagulant of the teat pack material were already explained, thus, an explanation thereof is omitted.

Further, the [Method for formation of the pack] and [Teat pack for lactating livestock] in the case when comprising (D) the release agent were explained in 1.[Production method -1 of (A) the base and (B) the coagulant], thus, an explanation thereof is omitted.

### 7.[Production method -4 of (A) the base and (B) the coagulant]

The teat pack material for lactating livestock of the present invention preferably comprises (G) an antifreezing agent in addition to (A) the base comprising the polymer latex and (B) the coagulant.

The production method of the teat pack material when the pack material for lactating livestock comprises (A) the base comprising the polymer latex, (D) the coagulant, and (G) the antifreezing agent is not specifically limited. At least about 9% of (G) the antifreezing agent per mass of the polymer latex can be comprised in one of (A) the latex or (B) the coagulant, and can be adjusted by the same adjustment method as stated above in the 1.[Production method -1 of (A) the base and (B) the coagulant] after blending (D) the release agent, (F) water and/or the water-soluble solvent and the additives in accordance with need.

The type, the blending amount and the like of (G) the antifreezing agent were already explained, thus, an explanation thereof is omitted.

Further, the [Method for formation of the pack] and [Teat pack for lactating livestock in the case when comprising (G) the antifreezing agent was explained in 1.[Production method -1 of (A) the base and (B) the coagulant], thus, an explanation thereof is omitted.

### EXAMPLES

Example 1 to Example 71 and Comparative example 1 to Comparative example 7 for verifying the effect of the examples are provided to specifically explain the present invention, but the present invention is not limited to these examples.

[(A) the polymer latex], [(B) the coagulant], [(C) the surfactant], [(D) the release agent] and [(F) water and/or the water-soluble solvent] were used in Example 1 to Example 71 and Comparative example 1 to Comparative example 7 as the raw materials for producing the teat pack. The common names and abbreviations thereof are as follows.

### Common names and abbreviations of [(A) the polymer latex]

- natural rubber latex (NR): ULACOL manufactured by Regitex Co., Ltd, solid component concentration of 62%, a viscosity of 50 mPa·s, and a minimum film-forming temperature of 10°C isoprene rubber latex (IR): Nipol ME manufactured by Zeon Corporation, a solid component concentration of 50%, a viscosity of 50 mPa·s, and a minimum film-forming temperature of 10°C
- butyl rubber latex (BUR) : JSR BUTYL065 manufactured by JSR Corporation, a solid component concentration of 50%, a viscosity of 32 mPa·s, and a minimum film-forming temperature of 10°C
- butadiene rubber latex (BR): JSR BR01 manufactured by JSR Corporation, a solid component concentration of 50%, a viscosity of 45 mPa·s, and a minimum film-forming temperature of 10°C
- styrene / butadiene latex (SB) : JSR SL552 manufactured by JSR Corporation, a solid component concentration of 50%, a 1:4 polymerization ratio of styrene and butadiene, a viscosity of 55 mPa·s, and a minimum film-forming temperature of 5°C
- urethane rubber latex (UR) : Superflex 460 manufactured by DSK Co., Ltd., a solid component concentration of 40%, a viscosity of 50 mPa·s, and a minimum film-forming temperature of 5°C
- nitrile rubber latex (NIR) : JSR N222SH manufactured by JSR Corporation, a solid component concentration of 50%, a viscosity of 85 mPa·s, and a minimum film-forming temperature of 5°C
- acrylic rubber latex (ACR): 2580 manufactured by Nissin Chemical Co., Ltd, a solid component concentration of 45%, a viscosity of 50 mPa·s, and a minimum film-forming temperature of 10°C
- vinyl acetate rubber latex (VA): A23J1-F2 manufactured by Nissin Chemical Co., Ltd, a solid component concentration of 45%, a viscosity of 2000 mPa·s, and a minimum film-forming temperature of 15°C
- ethylene vinyl acetate rubber latex (EVA): 4018 manufactured by Nissin Chemical Co., Ltd, a solid component concentration of 61%, a 1:1 polymerization ratio of ethylene and vinyl acetate, a viscosity of 300 mPa·s, and a minimum film-forming temperature of 10°C
- silicone rubber latex (SIR): KM-860A manufactured by Shin-Etsu Chemical Co., Ltd., a solid component concentration of 60%, a viscosity of 350 mPa·s, and a minimum film-forming temperature of -15°C
- fluororubber latex (FR): E-3705S21R manufactured by Daikin Industries Ltd., a solid component concentration of 50%, a viscosity of 300 mPa·s, and a minimum film-forming temperature of 10°C

Many of the aforementioned (A) polymer latexes having a grade such as a wide variety of solid contents (%), viscosities (mPa·s), pH, average particle sizes (nm), glass transition points (Tg (°C)), MFT (minimum film forming temperature (°C), acid value (KOH mg/g) are commercially available as emulsions with the dispersion medium as water. In the present invention, commercially available natural or synthetic rubber-based latex was purchased and used by physically adjusting so as to be suitable for a pack for lactating livestock teat.

### Common names and abbreviations of [(B) the coagulant]

In the present invention, one type selected from the group consisting of (a) the low molecular inorganic coagulant, (b) the polymer inorganic coagulant, (c) the polymer organic coagulant, (d) the acid, and (e) mixtures thereof was used as (B) the coagulant. Thereamong, the examples describe the common names and abbreviations of the polyvalent metal compound used as (a) the low molecular inorganic coagulant and the organic product which was used as (d) the acid.

### [Organic acid]

- lactic acid (LA): manufactured by Wako Pure Chemical Industries, Ltd.
- acetic acid (AA): manufactured by Wako Pure Chemical Industries, Ltd.
- propionic acid (PA): manufactured by Wako Pure Chemical Industries, Ltd.
- fumaric acid (FA): manufactured by Wako Pure Chemical Industries, Ltd.
- maleic acid (MAA): manufactured by Wako Pure Chemical Industries, Ltd.
- citric acid (CA): manufactured by Wako Pure Chemical Industries, Ltd.
- malic acid (MA): manufactured by Wako Pure Chemical Industries, Ltd.
- tartaric acid (TA): manufactured by Wako Pure Chemical Industries, Ltd.
- gluconic acid (GA): manufactured by Wako Pure Chemical Industries, Ltd.
- succinic acid (SA): manufactured by Wako Pure Chemical Industries, Ltd.

### [Polyvalent metal compound]

- calcium chloride (CC): manufactured by Wako Pure Chemical Industries, Ltd.
- magnesium chloride (MC): manufactured by Wako Pure Chemical Industries, Ltd.
- alum (AL): manufactured by Wako Pure Chemical Industries, Ltd.

### Common names and abbreviations of [(C) the surfactant]

### [Anionic surfactant]

- ammonium laurate (LAA): manufactured by Wako Pure Chemical Industries, Ltd.
- sodium lauryl sulfate (LSS): manufactured by Wako Pure Chemical Industries, Ltd.
- sodium dodecanesulfonate (SDS): manufactured by Wako Pure Chemical Industries, Ltd.

### [Cationic surfactant]

- tetrapropylammonium chloride (PAC): manufactured by Wako Pure Chemical Industries, Ltd.

### Common names and abbreviations of [(D) the release agent]

- polyether-modified silicone oil (PES): KF-351A manufactured by Shin-Etsu Chemical Co., Ltd., a viscosity of 70 mPa·s
- silicone oil (SO): KF-96 manufactured by Shin-Etsu Chemical Co., Ltd., a viscosity of 50 mPa·s
- Daifree (*): DF, fluorine-based release agent, external mold release type, water-based type
- Moldspat (**): MS, fluorine-based release agent, water type
   *) registered trade name of the fluorine-based release agent manufactured by Daikin Industries Ltd.
   **) registered trade name of the fluorine-based release agent manufactured by AGC Seimi Chemical Co., Ltd.

### Common names and abbreviations of [(F) water and/or the water-soluble solvent]

- water: WA
- ethanol: ET, manufactured by Wako Pure Chemical Industries, Ltd.
- isopropanol: IPA, manufactured by Wako Pure Chemical Industries, Ltd.
- butanol: BT, manufactured by Wako Pure Chemical Industries, Ltd.
- acetone: AC, manufactured by Wako Pure Chemical Industries, Ltd.

### [Solvent, raw materials, and the like used in the comparative examples]

- water: WA
- potassium alginate (20°C, 1% aqueous solution, and a viscosity of 300 mPa·sec): Alg-K, manufactured by Kimica Corporation
- sodium alginate (20°C, 1% aqueous solution, and a viscosity of 300 mPa·sec): Alg-Na, manufactured by Kimica Corporation
- calcium sulfate: calcium sulfate dihydrate manufactured by Wako Pure Chemical Industries, Ltd.

### Organic solvent solution of a polymer composition for preparing the teat pack composition

- a: 12 parts by mass of the solute poly urethane rubber dissolved in 88 parts by mass of the solvent tetrahydrofuran: [Table 7]
- b: 12 parts by mass of the solute vinyl acetate dissolved in 88 parts by mass of the solvent xylene: [Table 7]
- c: 9 parts by mass of the solute butyl rubber dissolved in 91 parts by mass of the solvent toluene: [Table 7]
- d:9 parts by mass of the solute butadiene rubber dissolved in 91 parts by mass of the solvent toluene: [Table 7]

### [Evaluation methods of the physical properties of the teat pack]

### 1.[Evaluation method of the adhesiveness]

After a dental silicone impression material ("Sofreliner Tough Supersoft" manufactured by Tokuyama Dental Corporation) was placed into a mold having the shape (inner diameter of 3 cmϕ and length of 4 cm) of the teat of dairy cattle, the impression material was cured, and the impression material was removed from the mold and a pseudo teat was made.

In Example 1 to Example 50, 40 g of the respective base and the coagulant were respectively filled within the cup (cylindrical container having an inner diameter of 6 cmϕ and a length of 4 cm). Next, holding the upper end of the aforementioned pseudo teat, a force was slowly applied from a state in contact with the base filled in the cup, 20% of the length of the pseudo teat tip was inserted and immediately pulled out. Next, the pack was produced in the vicinity of the pseudo teat by adhering (immersing) the entirety of the pseudo teat coated with the base in the coagulant by a method in the same manner. The film forming temperature was made to 37°C which is close to the temperature of the teat.

In Comparative example 1, the base was filled within the cup (a cylindrical container having an inner diameter of 6 cmϕ and a length of 4 cm). Next, holding the upper end of the aforementioned pseudo teat, a force was slowly applied from a state in contact with the base filled in the cup, 20% of the length of the pseudo teat tip was inserted and immediately pulled out to coat the base on the pseudo teat tip and then left standing. In Comparative example 2 to Comparative example 5, 40 g of the organic solvent solutions a to d of each polymer composition for preparing the teat pack composition were filled within the respective cups (cylindrical container having an inner diameter of 6 cmϕ and a length of 4 cm). Next, holding the upper end of the aforementioned pseudo teat, a force was slowly applied from a state in contact with the base filled in the cup, 20% of the length of the pseudo teat tip was inserted and immediately pulled out to coat the base on the pseudo teat tip and then left standing. In Comparative example 6 and Comparative example 7, the components were weighed within the cup for kneading so that the weight ratio of the powder components to the liquid components is 1:15, a spatula for kneading was used to knead until a uniform paste was obtained, and the obtained paste was filled within the cup. Next, holding the upper end of the aforementioned pseudo teat, a force was slowly applied from a state in contact with the base filled in the cup, the teat was inserted until it became hidden, was immediately pulled out, and the paste was cured (left standing for about 10 minutes).

The pack (pack produced in the pseudo teat tip) produced by the aforementioned method was left standing under the conditions of 23°C and a relative humidity of 50% with the teat side of the pseudo teat hanging downwards, the state of the teat pack was visually observed over time, and was evaluated by the following evaluation criteria.
A: The teat pack adhered with the pseudo teat, and completely adhered to the shape, so that gaps could not be visually recognized between the pack and the pseudo teat.
B: Gaps were visually recognized in the peripheral part of the teat pack, but the adhesiveness was maintained in the vicinity of the teat canal.
C: The teat pack was lifted up and peeling in the vicinity of the teat canal was visually recognized.

### 2.[Evaluation method of the durability]

After a dental silicone impression material ("Sofreliner Tough Supersoft" manufactured by Tokuyama Dental Corporation) was placed into a mold having the shape (inner diameter of 3 cmϕ and length of 4 cm) of the teat of dairy cattle, and the impression material was cured, the impression material was removed from the mold and the pseudo teat was made. The packs were respectively left standing for 15 minutes, 1 hour, 1 day, 2 days, 3 days, or 4 days under the conditions of a temperature of 23°C and a relative humidity of 50%RH. The durability test shown below was conducted on each of the packs. The actual pack usage site was considered, and in the durability test, the surface which impacts with the packs is the soil. The packs were set in a fatigue testing machine (E3000 manufactured by Instron Co., Ltd) and a load of 1 to 5 kgf/cm² was applied in order to add the load from the direction of the center axis of the pseudo teat. Note that, 3 kgf/cm² was set as the median of the load, and the amplitude was set to 2 kgf/cm². Further, the frequency was set to 1Hz and the cycle number was set to 100. After conducting the durability test, the conditions of the packs were visually observed, and the "durability" was evaluated based on the following evaluation criteria. Note that, if after three days, the evaluation was "C", no further tests were conducted. In the table, this is denoted as "-" (no evaluation).

### [Durability evaluation criteria]

The durability was evaluated by the following evaluation criteria.
A: Damage and the lifting up of the pack was not visually recognized.
B: While the pack was partially damaged, the vicinity of the teat canal was coated.
C: The entirety was damaged or was peeled.

### 3.[Evaluation method of the abrasion resistance]

After a dental silicone impression material ("Sofreliner Tough Supersoft" manufactured by Tokuyama Dental Corporation) was placed into a mold having the shape (inner diameter of 3 cmϕ and length of 4 cm) of the teat of dairy cattle, and the impression material was cured, the impression material was removed from the mold and the pseudo teat was made. The packs were formed in the vicinity of the pseudo teat in the same manner as the case of the evaluation of the "adhesiveness". Next, the packs were respectively left standing for 15 minutes, 1 hour, 1 day, 2 days, 3 days, or 4 days under the conditions of a temperature of 23°C and a relative humidity of 50%RH. The abrasion test shown below was conducted on each of the packs. The pseudo teat was fixed and immobilized by a jig above the teat canal and placed on a balance, and was scrubbed with 50 double scrubs in the horizontal direction by a brush (length of the bristles: 25 mm, material of the bristles: Nylon) in the vicinity of the teat canal. Then, the amplitude was set to about 5 cm and the load was set to about 1 kgf. After the abrasion test was conducted, the condition of the pack was observed visually, and the "abrasion resistance" was evaluated based on the following evaluation criteria. Note that, if after three days, the evaluation was "C", no further tests were conducted. In the table, this is denoted as "-" (no evaluation).

### [Abrasion resistance evaluation criteria]

The abrasion resistance was evaluated by the following evaluation criteria.
A: No exposure in the vicinity of the teat canal was visible.
B: Part of the teat canal was exposed.
C: The teat canal was completely exposed.

### 4.[Evaluation method of the number of bacteria]

Four days after the packs were attached in the evaluation of the aforementioned 2.[Evaluation method of the durability], the film was carefully peeled so that the contamination did not adhere to the inside of the pack after the durability test. An approximately 1 cm² portion corresponding to the teat canal which formed the packs was wiped with a swab (Promedia ST-25 manufactured by Elmex), and the swab was immersed in 10 mL of physiological saline. 1 mL of the solution therein was dripped in a culture medium (aerobic bacteria culture, 6400AC, manufactured by 3M), and was cultured at 37°C for 48 hours. After culturing, the number of colonies in the culture was counted, it was assumed that one bacteria forms one colony, and the number of bacteria in the aforementioned 10 mL of physiological saline was calculated (the number of colonies x 10 = number of bacteria) from the number of colonies. The number of bacteria which was sought was evaluated according to the following criteria. Generally, if the number of bacteria was less than 1000/cm², it was deemed that cleanliness was maintained.

Note that, the number of external bacteria (the number of bacteria in soil) was roughly 10000/cm² or more although there was variation.

### [Number of bacteria evaluation criteria]

The number of bacteria was evaluated by the following evaluation criteria.
A: 1000/cm² or less: the number of bacteria was few, and the inside of the pack was maintained in a clean condition.
B: 1000/cm² or more to less than 10000/cm²: the number of bacteria was somewhat large, but was less than the number of external bacteria.
C: 10000/cm² or more: the number of bacteria was large, so that the reduction effect on the number of bacteria due to the pack could not be verified.

### 5.[Evaluation method of ease of pack removal]

The pack was produced in the same manner as the aforementioned 1.[Evaluation method of the adhesiveness], and was left standing under the conditions of 23°C and a relative humidity of 50% with the teat side of the pseudo teat hanging downwards. Whether the pack can be removed cleanly when the entirety of the pseudo teat was wiped with a cotton cloth (20 cm²) was evaluated.

The ease of pack removal was evaluated by the following evaluation criteria.
A: The pack could be easily peeled when wiped one time.
B: The pack could not be peeled one time, but could be peeled if wiped two or three times.
C: The pack could not be peeled even if wiped two or three times.

### 6.[Evaluation method of the adhesiveness to the teat coated with oil and fat]

After a dental silicone impression material ("Sofreliner Tough Supersoft" manufactured by Tokuyama Dental Corporation) was placed into a mold having the shape (inner diameter of 3 cmϕ and length of 4 cm) of the teat of dairy cattle, and the impression material was cured, the impression material was removed from the mold and the pseudo teat was made. The commercially available milk (milk fat content 3.6%) and the teat pack materials were respectively filled in the 40 g cups (a cylindrical container having an inner diameter of 6 cmϕ and a length of 4 cm). Next, holding the aforementioned upper end of the pseudo teat, 50% of the length of the pseudo teat tip was inserted in the milk filled in the cup and removed once, and then, the teat pack material was adhered to the pseudo teat tip by immediately inserting 20% of the length of the pseudo teat tip in the teat pack material and immediately pulling out. Then, the pack was produced in the vicinity of the pseudo teat by naturally drying for a while (about ten minutes). Note that, the film formation temperature was set to 37°C which is close to the temperature of the teat. Note that, coating the teat pack material to the pseudo teat on which milk was adhered is assumed to be the case when the teat pack material is made to adhere to the teat of lactating livestock on which milk was adhered.

The pack (pack produced in the pseudo teat tip) produced by the aforementioned method was left standing under the conditions of 23°C and a relative humidity of 50% with the teat side of the pseudo teat hanging downwards, the state of the teat pack was visually observed over time, and was evaluated by the following evaluation criteria.
A: The pack completely adhered to the pseudo teat, gaps were not visually recognized between the pack and the pseudo teat, and no falling off of the pack occurred.
B: While gaps were visually recognized in the peripheral part of the pack, the vicinity of the teat canal was adhered.
C: The pack lifted up and was peeled in the vicinity of the teat canal.

### 7.[Evaluation method of the coatability on the teat coated with oil and fat]

The coatability was evaluated visually by the following criteria when the teat pack materials of Example 37 to Example 71 were respectively adhered to the pseudo teat to which the commercially available milk adhered in the evaluation of the aforementioned 6.[Evaluation method of adhesiveness to the teat coated with oil and fat].
3: Was uniformly coatable.
2: Slight unevenness, but was almost uniformly coatable.
1: Unevenness was large, but the entire surface was coatable.
0: Unevenness was remarkably large, so that there were portions which were not coatable.

### 8.[Evaluation method of the ability to maintain on the teat coated with oil and fat]

The teat pack materials of Example 37 to Example 50 were adhered to the teat of the actual dairy cattle. The dairy cattle for use in the tests were selected from those whose teats were greasy due to milk and oil and fat on the teat skin. Note that, the pack was attached to the dairy cattle soon after the milking operation. The teat pack material was adhered to two cows for milking (there are four teats per animal, therefore a total of 8 teats). Namely, 40 g of the teat pack materials of Example 37 to Example 50 was filled in the cup (a cylindrical container having an inner diameter of 6 cmϕ and a length of 4 cm), and next, the teat pack material was adhered to the teat and the pack was formed by immersing 20% of the length of the teat in the teat pack material and pulling out. Then, the cows were put to grazing, verification of the pack attachment state was performed after 1 hour, after 6 hours, and after 12 hours, and the ability to maintain the pack was evaluated by the following criteria. Note that, the floor of the grazing is soil.

The ability to maintain the pack on the teat coated with oil and fat evaluation criteria:
A: 6 or more of the 8 packs attached remained attached
B: 4 to 5 teat packs of the 8 packs attached remained attached
C: 3 teat packs or less of the 8 packs attached remained attached.

### 9.[Evaluation method of the adhesive force]

The entire surfaces of two PET films (width of 20 mm, length of 100 mm, and thickness of 0.1 mm) were coated so that the teat pack materials manufactured in Example 51 to Example 71 became a thickness of 0.1 mm. After leaving at 23°C and 30 minutes had elapsed after coating, the film (cured teat pack material) laminated on the two PET films was stuck together in a condition in which a pressure of 1N was applied so as to be brought in contact. Then, the film was stripped off by a tensile testing machine under the conditions of stripping in the 180° direction at a speed of 200 mm/min, and the value obtained by dividing the peeling force (the load (N) required for stripping) at this time by 2 is defined as the adhesive force (N/10 mm). Note that, the measurement of the adhesive force was performed at 23°C.

### 10.[Evaluation method of the ability to maintain on the teat in an approach state]

40 g of the teat pack materials manufactured in Example 51 to Example 71 was filled within the cup (a cylindrical container having an inner diameter of 6 cmϕ and a length of 4 cm). Next, approximately 20% of the length of the posterior teat of an actual dairy cattle was immersed in the teat pack material, the teat pack material was attached to the teat by pulling out, and the pack was formed. The dairy cattle used for the test were selected from cows whose posterior teats were in contact or in close proximity. Note that, the pack was attached to the dairy cattle soon after the milking operation. The teat pack material was adhered to four dairy cattle (there are two rear teats per animal, therefore a total of 8 teats). Then, the cows were put to grazing, verification of the pack attachment state was performed after 1 hour, after 6 hours, and after 12 hours, and the ability to maintain the pack was evaluated by the following criteria. Note that, the floor of the grazing is soil.
A: 6 or more of the 8 packs attached remained attached
B: 4 to 5 teat packs of the 8 packs attached remained attached
C: 3 teat packs or less of the 8 packs attached remained attached

### 11.[Evaluation method of the presence of adhesion]

In the aforementioned 10.[Evaluation method of the ability to maintain on the teat in an approach state], the posterior teat of the dairy cattle to which the teat packs manufactured in Example 51 to Example 71 were attached were observed after one hour of grazing, and the presence of adhesion of the packs was evaluated visually by the following criteria.
A: The adhesion of the pack was completely not recognized.
B: A major portion was not adhered, but the adhesion to a part of the pack was recognized.
C: The adhesion of the pack was largely recognized.

### [Example 1]

40g of (A) natural rubber latex (NR) was weighed into the container and set as the base. Further, 40 g of (B) lactic acid (LA) was weighed into the container and set as the coagulant. The base and the coagulant were used to form the packs, and the evaluations of the "adhesiveness", the "durability", the "abrasion resistance", the "number of bacteria", and the "ease of pack removal" were performed.

### [Example 2 to Example 25]

With the exception of using (A) the bases and (B) the coagulants shown in Table 1 to Table 2, the packs were formed and the evaluations of "adhesiveness", the "durability", the "abrasion resistance", the "number of bacteria", and the "ease of pack removal" in the same manner as Example 1

### [Example 45 and Example 46]

With the exception of using (A) the bases and (B) the coagulants shown in Table 3, the packs were formed and the evaluations of the "adhesiveness to the teat coated with oil and fat", the "adhesiveness", the "coatability on the teat coated with oil and fat", the "ability to maintain on the teat coated with oil and fat", the "durability", the "number of bacteria", and the "ease of pack removal" were performed in the same manner as Example 1. Note that, 40 g of commercially available milk (milk fat content 3.6%) was weighed into the container.

### [Example 64 and Example 65]

With the exception of using (A) the bases and (B) the coagulants shown in Table 5, the packs were formed and the evaluations of the "ability to maintain on the teat in an approach state", "the presence of adhesion", the "durability", the "coatability on the teat coated with oil and fat", and the "ease of pack removal" were performed in the same manner as Example 1. Note that, 40 g of commercially available milk (milk fat content 3.6%) was weighed into the container.

### [Example 26]

40 g of (A) natural rubber latex (NR) was weighed into the container and set as the base. 100 g of lactic acid (LA) was added to 500 g of water (WA), and agitated for 30 minutes to prepare the coagulant.

40 g from among the prepared coagulant was weighed into the container. The base and the coagulant were used to form the packs, and the evaluations of the "adhesiveness", the "durability", the "abrasion resistance", the "number of bacteria", and the "ease of pack removal" were performed.

### [Example 27 to Example 36]

With the exception of making the configuration of (A) the base and (B) the coagulant to the amounts shown in Table 2, the packs were formed and the evaluations of the "adhesiveness", the "durability", "abrasion resistance", the "number of bacteria", and the "ease of pack removal" were performed in the same manner as Example 26.

### [Example 47 to Example 50]

With the exception of making the configuration of (A) the base and (B) the coagulant to the amounts shown in Table 3, the packs were formed and the evaluations of the "adhesiveness to the teat coated with oil and fat", the "adhesiveness", the "coatability on the teat coated with oil and fat", the "ability to maintain on the teat coated with oil and fat", the "durability", the "number of bacteria", and the "ease of pack removal" were performed in the same manner as in Example 26. Note that, 40 g of commercially available milk (milk fat content 3.6%) was weighed into the container.

### [Example 51 to Example 63 and Example 66 to Example 69]

With the exception of making the configuration of (A) the base and (B) the coagulant to the amounts shown in Table 4 and Table 5, the packs were formed and the evaluations of the "ability to maintain on the teat in an approach state", "the presence of adhesion", the "durability", the "coatability on the teat coated with oil and fat", and the "ease of pack removal" were performed in the same manner as Example 26. Note that, 40 g of commercially available milk (milk fat content 3.6%) was weighed into the container.

### [Example 37]

0.8 g of ammonium laurate was added to 400 g of (A) natural rubber latex (NR) and agitated for 30 minutes to prepare the base. 40 g from among the prepared base was weighed into the container. Further, 40 g of (B) lactic acid (LA) was weighed into the container and set as the coagulant. The base and the coagulant were used to form the packs, and the evaluations of the "adhesiveness to the teat coated with oil and fat", the "adhesiveness", the "coatability on the teat coated with oil and fat", the "ability to maintain on the teat coated with oil and fat", the "durability", the "number of bacteria", and the "ease of pack removal" were performed. Note that, 40 g of commercially available milk (milk fat content 3.6%) was weighed into the container.

### [Example 38, Example 43, and Example 44]

With the exception of making the configuration of (A) the base and (B) the coagulant to the amounts shown in Table 3, the packs were formed and the evaluations of the "adhesiveness to the teat coated with oil and fat", the "adhesiveness", the "coatability on the teat coated with oil and fat", the "ability to maintain on the teat coated with oil and fat", the "durability", the "number of bacteria", and the "ease of pack removal" were performed in the same manner as Example 37. Note that, 40 g of commercially available milk (milk fat content 3.6%) was weighed into the container.

### [Example 39]

0.8 g of ammonium laurate was added to 400 g of (A) natural rubber latex (NR) ((C) surfactant content in the base: 0.2 mass%), and agitated for 30 minutes and the base was prepared. 40 g from among the prepared bases was weighed into the container. Further, 100 g of lactic acid (LA) was added in 500 g of water (WA), and agitated for 30 minutes and the coagulant was prepared. 40 g from among the prepared coagulants was weighed into the container. The base and the coagulant were used to form the packs, and the evaluations of the "adhesiveness to the teat coated with oil and fat", the "adhesiveness", the "coatability on the teat coated with oil and fat", the "ability to maintain on the teat coated with oil and fat", the "durability", the "number of bacteria", and the "ease of pack removal" were performed. Note that, 40 g of commercially available milk (milk fat content 3.6%) was weighed into the container.

### [Example 40 to Example 42]

With the exception of making the configuration of (A) the base and (B) the coagulant to the amounts shown in Table 3, the packs were formed and the evaluations of the "adhesiveness to the teat coated with oil and fat", the "adhesiveness", the "coatability on the teat coated with oil and fat", the "ability to maintain on the teat coated with oil and fat", the "durability", the "number of bacteria", and the "ease of pack removal" were performed in the same manner as Example 39. Note that, 40 g of commercially available milk (milk fat content 3.6%) was weighed into the container.

### [Example 70 and Example 71]

With the exception of making the configuration of (A) the base and (B) the coagulant to the amounts shown in Table 5, the pack were formed and the evaluations of the "ability to maintain on the teat in an approach state", "the presence of adhesion", the "durability", the "coatability on the teat coated with oil and fat", and the "ease of pack removal" were performed in the same manner as Example 26. Note that, 40 g of commercially available milk (milk fat content 3.6%) was weighed into the container.

### [Comparative Example 1]

40 g of a natural rubber latex was weighed into the container and set as the base. The packs were formed without using (B) the coagulant at all, and the evaluations of the "adhesiveness", the "durability", the "abrasion resistance", the "number of bacteria", and the "ease of pack removal" were performed. The configuration of Comparative example 1 is shown in Table 6.

### [Comparative example 2 to Comparative example 5]

The organic solvent solution of a polymer composition for preparing the teat pack compositions was used as is as the base. The base was used to form the packs, and the evaluations of the "adhesiveness", the "durability", the "abrasion resistance", the "number of bacteria", and the "ease of pack removal" were performed. The configurations of Comparative example 2 to Comparative example 5 are shown in Table 7.

### [Comparative Example 6]

20.0 g of sodium alginate and 20.0 g of calcium sulfate were respectively charged in a small mixer (food mixer manufactured by Iwantani Corporation) agitated for five minutes, and a uniform powder component was obtained. Further, 600 g of distilled water was prepared as the liquid component. The powder components and the liquid components were used to form the packs, and the evaluations of the "adhesiveness", the "durability", the "abrasion resistance", the "number of bacteria", and the "ease of pack removal" were performed. The configuration of Comparative example 6 is shown in Table 8.

### [Comparative Example 7]

With the exception of making the configuration of the powder component to the amount shown in Table 8, the pack was formed and the evaluations of the "adhesiveness", the "durability", the "abrasion resistance", the "number of bacteria", and the "ease of pack removal" were performed in the same manner as Comparative example 6. The configuration of Comparative example 7 is shown in Table 8.

The configurations of the teat pack material for lactating livestock of Example 1 to Examples 71 are shown in Table 1 to Table 5, and the configurations of the teat pack material for lactating livestock of Comparative examples 1 to 7 are shown in Tables 6 to 8. Further, the evaluation results of the "adhesiveness", the "durability", the "abrasion resistance", the "number of bacteria", the "ease of pack removal", the "adhesiveness to the teat coated with oil and fat", the "coatability on the teat coated with oil and fat", the "ability to maintain on the teat in an approach state", and the "presence of adhesion" in Examples 1 to 71 and Comparative examples 1 to 7 are respectively shown in Table 9 to Table 12 and Table 13.

**[Table 1]**

| | Base | | | | | | | | | | | | | Coagulant |
|---|---|---|---|---|---|---|---|---|---|---|---|---|---|---|
| | (A) Latex | | | | | | | | | | | | | (B) Acid |
| | NR | IR | BUR I | BR | SB | UR | NIR | CR | ACR | VA | EVA | SIR | FR | LA |
| Example 1 | 100 | | | | | | | | | | | | | 100 |
| Example 2 | | 100 | | | | | | | | | | | | 100 |
| Example 3 | | | 100 | | | | | | | | | | | 100 |
| Example 4 | | | | 100 | | | | | | | | | | 100 |
| Example 5 | | | | | 100 | | | | | | | | | 100 |
| Example 6 | | | | | | 100 | | | | | | | | 100 |
| Example 7 | | | | | | | 100 | | | | | | | 100 |
| Example 8 | | | | | | | | 100 | | | | | | 100 |
| Example 9 | | | | | | | | | 100 | | | | | 100 |
| Example 10 | | | | | | | | | | 100 | | | | 100 |
| Example 11 | | | | | | | | | | | 100 | | | 100 |
| Example 12 | | | | | | | | | | | | 100 | | 100 |
| Example 13 | | | | | | | | | | | | | 100 | 100 |

**[Table 2]**

| | Base | Coagulant | | | | | | | | | | | | | | | | | |
|---|---|---|---|---|---|---|---|---|---|---|---|---|---|---|---|---|---|---|---|
| | | Essential component | | | | | | | | | | | | | Additive component | | | | |
| | (A) Latex | (B) Acid | | | | | | | | | | (B) Metal salt | | | (F) Water / water-soluble solvent | | | | |
| | NR | LA | AA | PA | FA | MAA | CA | MA | TA | GA | SA | CC | MC | AL | WA | ET | IPA | BT | AC |
| Example 14 | 100 | | 100 | | | | | | | | | | | | | | | | |
| Example 15 | 100 | | | 100 | | | | | | | | | | | | | | | |
| Example 16 | 100 | | | | 100 | | | | | | | | | | | | | | |
| Example 17 | 100 | | | | | 100 | | | | | | | | | | | | | |
| Example 18 | 100 | | | | | | 100 | | | | | | | | | | | | |
| Example 19 | 100 | | | | | | | 100 | | | | | | | | | | | |
| Example 20 | 100 | | | | | | | | 100 | | | | | | | | | | |
| Example 21 | 100 | | | | | | | | | 100 | | | | | | | | | |
| Example 22 | 100 | | | | | | | | | | 100 | | | | | | | | |
| Example 23 | 100 | | | | | | | | | | | 100 | | | | | | | |
| Example 24 | 100 | | | | | | | | | | | | 100 | | | | | | |
| Example 25 | 100 | | | | | | | | | | | | | 100 | | | | | |
| Example 26 | 100 | 100 | | | | | | | | | | | | | 500 | | | | |
| Example 27 | 100 | | | | | | | | | | | 100 | | | 500 | | | | |
| Example 28 | 100 | 100 | | | | | | | | | | | | | | 500 | | | |
| Example 29 | 100 | | | | | | | | | | | 100 | | | | 500 | | | |
| Example 30 | 100 | 100 | | | | | | | | | | | | | | | 500 | | |
| Example 31 | 100 | 100 | | | | | | | | | | | | | | | | 500 | |
| Example 32 | 100 | 100 | | | | | | | | | | | | | | | | | 500 |
| Example 33 | 100 | 100 | | | | | | | | | | | | | 1000 | | | | |
| Example 34 | 100 | 100 | | | | | | | | | | | | | 2000 | | | | |
| Example 35 | 100 | 100 | | | | | | | | | | | | | | 1000 | | | |
| Example 36 | 100 | 100 | | | | | | | | | | | | | | 2000 | | | |

**[Table 3]**

| | Teat pack material | | | | | | | | | Surfactant content in the base |
|---|---|---|---|---|---|---|---|---|---|---|
| | Base | | | | | Coagulant | | | | |
| | (A) Latex | (C) Surfactant | | | | (B) Acid / Metal salt | | (F) Water / water-soluble solvent | | |
| | NR | LAA | LSS | SDS | PAC | LA | CC | WA | ET | |
| Example 37 | 4000 | 8 | | | | 100 | | | | 0.2 |
| Example 38 | 4000 | 8 | | | | | 100 | | | 0.2 |
| Example 39 | 4000 | 8 | | | | 100 | | 500 | | 0.2 |
| Example 40 | 4000 | 8 | | | | 100 | | | 500 | 0.2 |
| Example 41 | 4000 | 8 | | | | | 100 | 500 | | 0.2 |
| Example 42 | 4000 | 8 | | | | | 100 | | 500 | 0.2 |
| Example 43 | 4000 | 0.8 | | | | 100 | | | | 0.02 |
| Example 44 | 4000 | 680 | | | | 100 | | | | 15 |
| Example 45 | 4000 | | | | | 100 | | | | 0 |
| Example 46 | 4000 | | | | | | 100 | | | 0 |
| Example 47 | 4000 | | | | | 100 | | 500 | | 0 |
| Example 48 | 4000 | | | | | 100 | | | 500 | 0 |
| Example 49 | 4000 | | | | | | 100 | 500 | | 0 |
| Example 50 | 4000 | | | | | | 100 | | 500 | 0 |

**[Table 4]**

| | Teat pack material | | | | | | | | | Adhesive force (N/10mm) |
|---|---|---|---|---|---|---|---|---|---|---|
| | Base | | | | | Coagulant | | | | |
| | (A) Latex | (D) Release agent | | | | (B) Acid / Metal salt | | (F) Water / water-soluble solvent | | |
| | NR | PES | SO | DF | MS | LA | CC | WA | ET | |
| Example 51 | 2000 | 100 | | | | 1800 | | | | 0.13 |
| Example 52 | 2000 | 100 | | | | | 1800 | | | 0.12 |
| Example 53 | 2000 | 100 | | | | 300 | | 1500 | | 0.13 |
| Example 54 | 2000 | 100 | | | | 300 | | | 1500 | 0.13 |
| Example 55 | 2000 | 100 | | | | | 300 | 1500 | | 0.12 |
| Example 56 | 2000 | 100 | | | | | 300 | | 1500 | 0.12 |

**[Table 5]**

| | Teat pack material | | | | | | | | | | Adhesive force (NI10mm) |
|---|---|---|---|---|---|---|---|---|---|---|---|
| | Base | | | | | | Coagulant | | | | |
| | (A) Latex | (C) Surfactant | (D) Release agent | | | | (B) Acid / Metal salt | | (F) Water / water-soluble solvent | | |
| | NR | LAA | PES | SO | DF | MS | LA | CC | WA | ET | |
| Example 57 | 2000 | | 100 | | | | 1800 | | | | 0.09 |
| Example 58 | 2000 | | 100 | | | | | 1800 | | | 0.08 |
| Example 59 | 2000 | | 100 | | | | 300 | | 1500 | | 0.09 |
| Example 60 | 2000 | | 100 | | | | 300 | | | 1500 | 0.08 |
| Example 61 | 2000 | | 100 | | | | | 300 | 1500 | | 0.09 |
| Example 62 | 2000 | | 100 | | | | | 300 | | 1500 | 0.08 |
| Example 63 | 2000 | | 1 | | | | 1800 | | | | 0.6 |
| Example 64 | 2000 | | | | | | 1800 | | | | 1.4 |
| Example 65 | 2000 | | | | | | | 1800 | | | 1.5 |
| Example 66 | 2000 | | | | | | 300 | | 1500 | | 1.4 |
| Example 67 | 2000 | | | | | | 300 | | | 1500 | 1.5 |
| Example 68 | 2000 | | | | | | | 300 | 1500 | | 1.4 |
| Example 69 | 2000 | | | | | | | 300 | | 1500 | 1.4 |
| Example 70 | 2000 | 4 | 100 | | | | | 300 | 1500 | | 0.06 |
| Example 71 | 2000 | 4 | 100 | | | | | 300 | | 1500 | 0.07 |

**[Table 6]**

| | Base | Coagulant |
|---|---|---|
| | (A) Latex | (B) Acid / Metal salt |
| | NR | |
| Comparative Example 1 | 100 | - |

**[Table 7]**

| | Teat pack material | Solute (parts by mass) | Solvent (parts by mass) |
|---|---|---|---|
| Comparative Example 2 | a | Urethane rubber (12) | Tetrahydrofuran (88) |
| Comparative Example 3 | b | Vinyl acetate (12) | Xylene (88) |
| Comparative Example 4 | c | Butyl rubber (9) | Toluene (91) |
| Comparative Example 5 | d | Butadiene rubber (9) | Toluene (91) |

**[Table 8]**

| Powder / liquid type | Teat pack material | | | |
|---|---|---|---|---|
| | Powder component | | | Liquid component |
| | Polysaccharide polyelectrolyte | | Polyvalent metal compound | Water |
| | Alq-Na | Alq-K | Calcium sulfate | Distilled water |
| Comparative Example 6 | 100 | | 100 | 200 |
| Comparative Example 7 | | 100 | 100 | 200 |

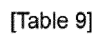

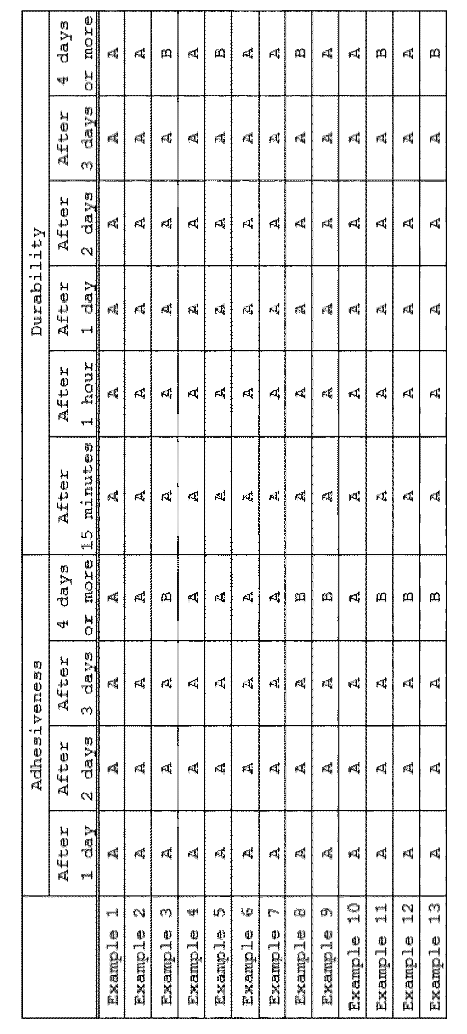

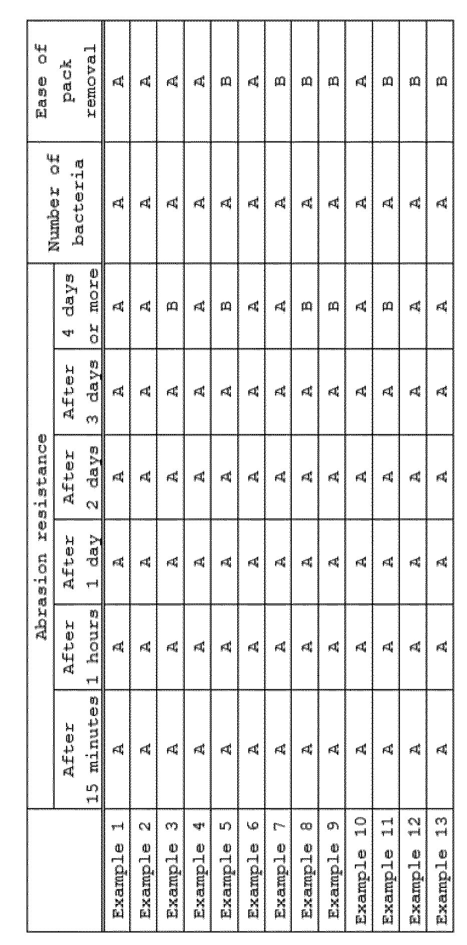

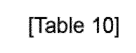

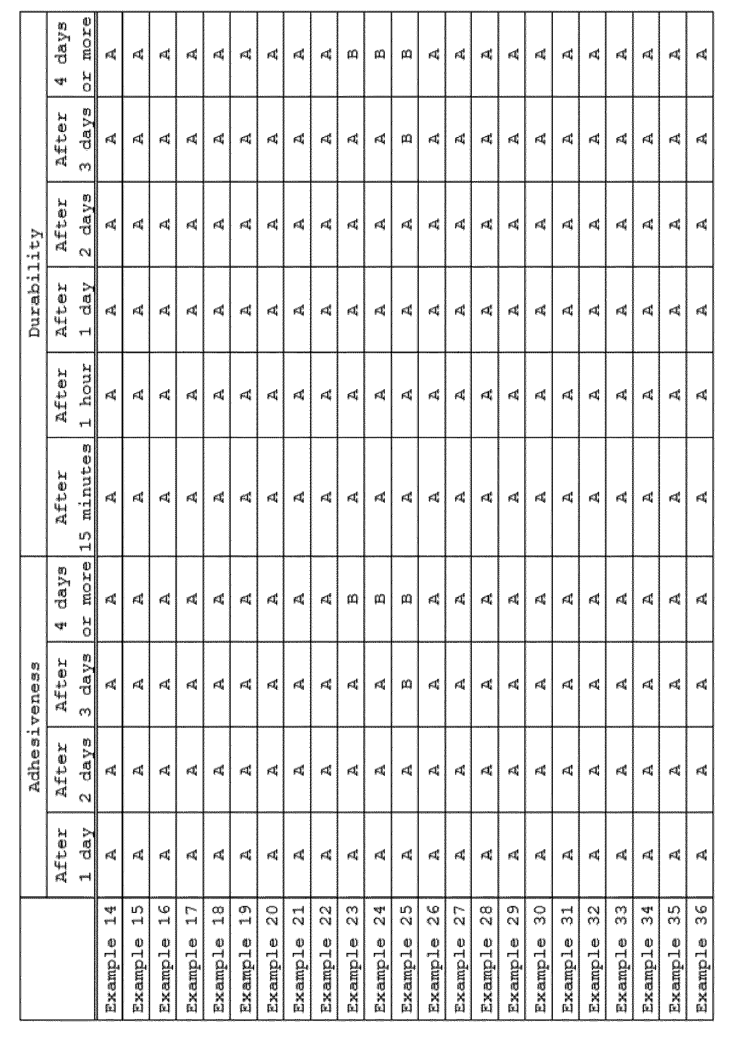

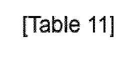

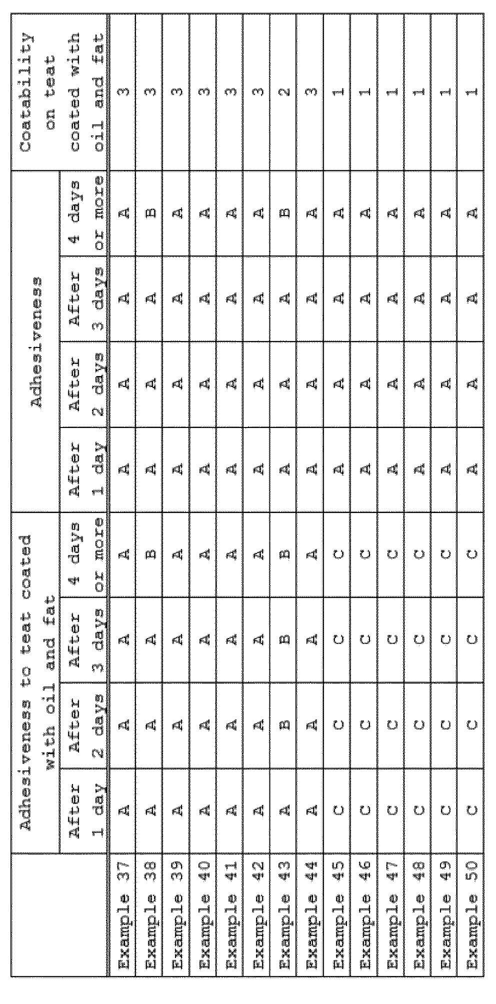

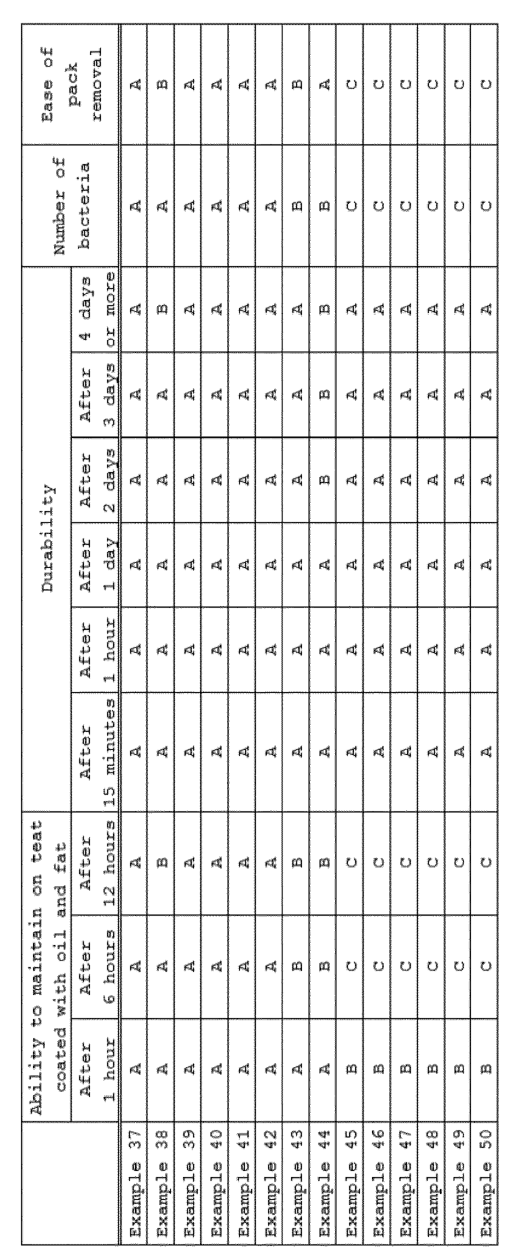

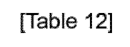

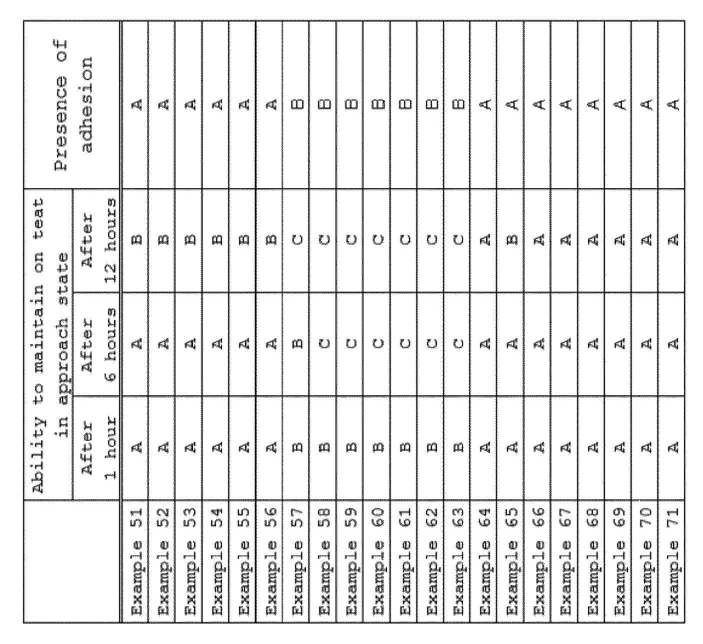

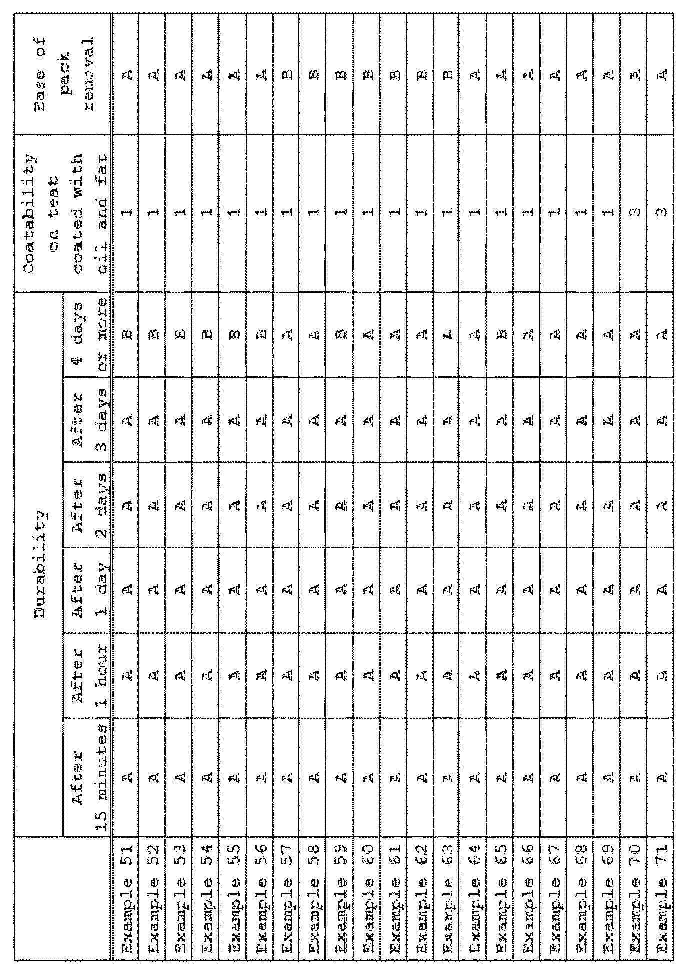

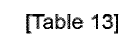

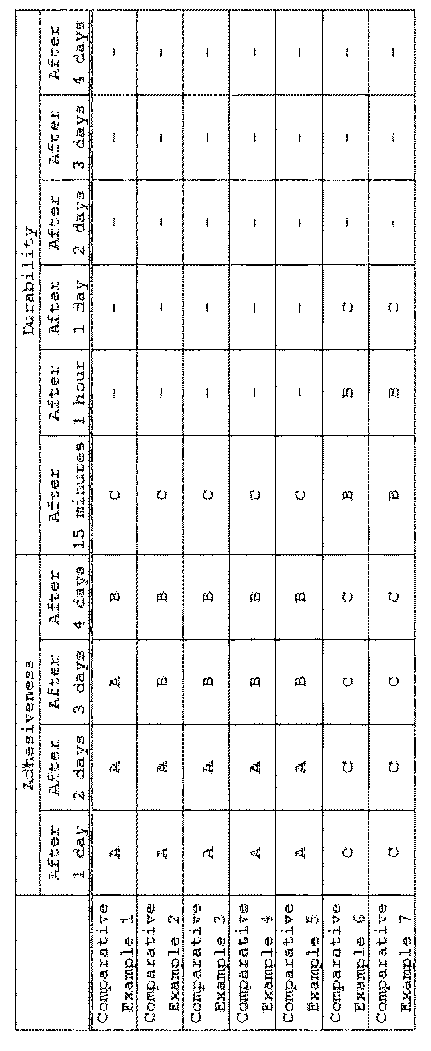

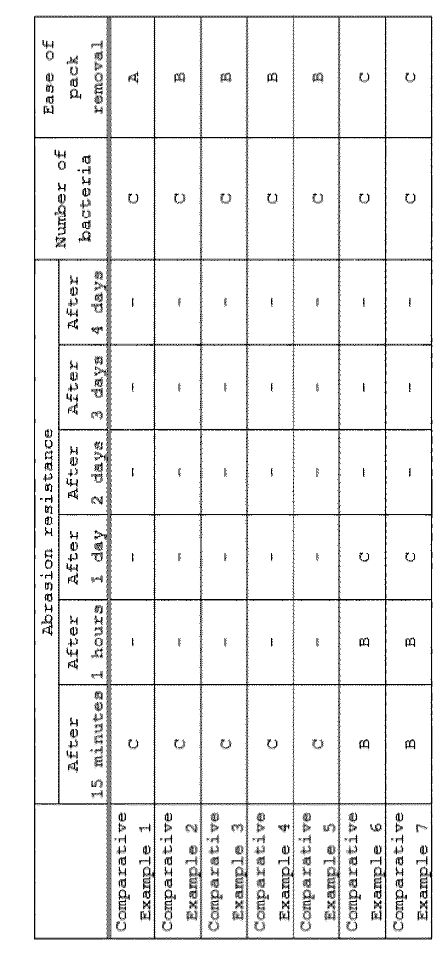

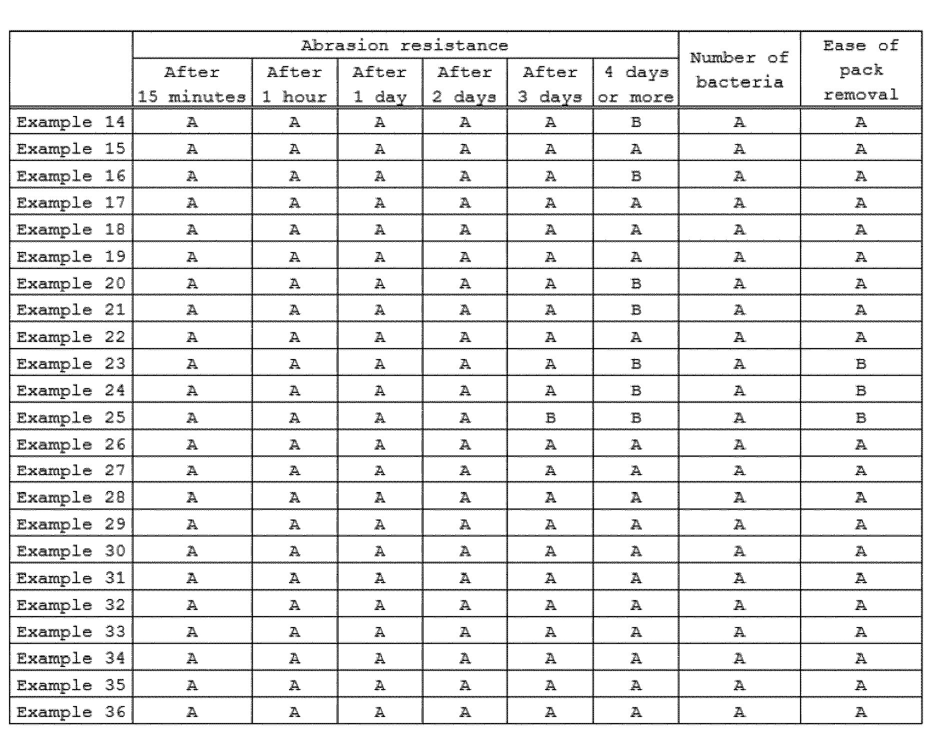

### [Discussion]

In Example 1 to Example 36, the conditions were adjusted in order to saitsfy all the requirements of the present invention, but in any case, the coating film could be obtained in which the adhesiveness, and the durability and the abrasion resistance were high. The number of bacteria was sufficiently low and the pack was easily removed. When a surfactant was added to the pack material, a satisfactory coatability onto the teat coated with oil and fat could be obtained, and when a release agent was added to the pack material, a satisfactory ability to maintain on the teat in a approach state could be obtained.

In Example 37 to Example 42, the conditions were adjusted in order to satisfy all the requierements of the present invention, but in any case, with an ordinary teat, the coating film could be obtained in which the coatability on the teat coated with oil and fat was good and the adhesiveness was high. The pack is a uniform coating film and the pack was easily removed. In Examples 37 to Examples 42, a uniform pack could be produced instantaneously, thus, the durability test after 15 minutes was withstood for 4 days or more. In addition, it is understood that the present invention brings about an excellent effect in the physical properties of all of the "adhesiveness to the teat coated with oil and fat", the "adhesiveness", the "coatability to the teat coated with oil and fat", the "ability to maintain on the teat coated with oil and fat", the "bacterial test" and the "ease of pack removal". It was demonstrated thereby that the aforementioned excellent results were brought about by virtue of a synergistic effect due to using both (C) the surfactant and the acid and/or the metal salt (low molecular inorganic coagulant) as (B) the coagulant. On the other hand, in Example 43, the content of (C) the surfactant is low, and only the acid (LA) was used as (B) the coagulant, and a polyvalent metal compound was not used, and thus, it was demonstrated to be inferior in several points such as the "adhesiveness to the teat coated with oil and fat", the "adhesiveness", the "coatability to the teat coated with oil and fat", the "ability to maintain on the teat coated with oil and fat", the "bacterial test" and the "ease of pack removal" when compared to Examples 37 to 42. Further, Examples 45 to 50 do not use (C) the surfactant, and in the so-called control tests, were obviously inferior in several points such as the "adhesiveness to the teat coated with oil and fat", the "coatability to the teat coated with oil and fat", the "ability to maintain on the teat coated with oil and fat", the "bacterial test" and the "ease of pack removal" compared to the other examples.

In Examples 51 to 71, the pack was formed instantaneously when using the acid and/or the metal salt (low molecular inorganic coagulant) as (B) the coagulant, and there were no breaks or peeling in the pack even ten minutes after the pack formation. Furthermore, the surfactant (LAA) was used in Examples 70 and 71, and it is understood that a uniform pack can be formed in which the coatability to the teat to which milk is adhered and is greasy (the teat coated with oil and fat) is good regardless of the state of the teat. From the comparison of Examples 51 to 56 and Examples 66 to 71, it is understood that for the teat pack material to be stored by separating into (A) the base and (B) the coagulant, the adhesive force between the packs becomes lower than when adding (D) the release agent to (B) the coagulant, and as a result, the ability to maintain on the teat in an approach state further increases.

With respect thereto, Comparative example 1 is an example in which (B) the coagulant selected from the group consisting of the low molecular inorganic coagulant, polymer inorganic coagulant, polymer organic coagulant, acid, and mixtures thereof were not used at all, but the pack material became dry and it required about 30 minutes for the pack to form, thus, the pack did not form at a point in time 15 minutes after coating the pack composition, the pack material dissipated during the durability test and during the abrasion resistance test and could not withstand the durability test and the abrasion test.

Comparative example 2 to Comparative example 5 are examples in which the organic solvent solution of a polymer composition for preparing the teat pack composition was used as the base, in accordance with Patent Document 3, but the pack material became dry and it required about 30 minutes for the pack to form, thus, the pack did not form at a point in time 15 minutes after coating the pack composition, and could not withstand the durability test and the abrasion test.

Comparative example 6 and Comparative example 7 are examples when applying the composition which uses an alginate salt, in accordance with Patent Document 4, but numerous air bubbles were mixed during kneading and the viscosity of the kneaded material was not uniform, thus, the composition becomes cracked in less than one day, and it is understood that the adhesiveness, the durability, and the abrasion resistance of the pack are remarkably low.

## Claims

1. Composition for coating the entire surface of a teat of milk producing livestock by forming a film which adheres closely to a teat of milk-producing livestock such composition comprising at least,
(A) a polymer latex, wherein (A) the polymer latex is an emulsion obtained by dispersing a natural or a synthetic rubber, or a synthetic polymer as a dispersoid in a dispersion medium mainly containing water and
(B) a coagulant, wherein (B) the coagulant dissociates a divalent or more metal ion within the dispersion medium, wherein (B) the coagulant is at least one type selected from citric acid, lactic acid, acetic acid, malic acid, fumaric acid, maleic acid, tartaric acid, gluconic acid, succinic acid, propionic acid, oxalic acid, benzoic acid or salts thereof.

2. Composition according to claim 1, wherein the teat pack material for lactating livestock further comprises at least one type selected from (C) a surfactant, (D) a release agent, (E) a water-soluble polymer, (F) water and/or a water-soluble solvent and (G) an antifreezing agent.

3. Composition according to claim 2, wherein (C) the surfactant is an anionic surfactant.

4. Composition according to claim 2, wherein (D) the release agent is compound comprising a -CF₃, -CF₂, -CH₃, or -CH₂ group.

5. Composition according to claim 2, wherein (E) the water-soluble polymer is at least one type selected from methyl cellulose, carboxymethyl cellulose, hydroxyethyl cellulose, casein, polyvinyl alcohol, polyvinylpyrrolidone, polyvinyl methyl ether, polyacrylic acid, polymethacrylic acid, polyacrylamide, polyethylene oxide and polyethyleneimide.

6. Composition according to claim 2, wherein (G) the antifreezing agent is at least one type selected from the group consisting of ethylene glycol, propylene glycol, glycerin, alcohols, potassium acetate and calcium chloride.

7. A kit for forming a pack which adheres to the teat of milk producing livestock, comprising (i) a base comprising at least (A) a polymer latex housed in a predetermined container 3, wherein (A) the polymer latex is an emulsion obtained by dispersing a natural or a synthetic rubber, or a synthetic polymer as a dispersoid in a dispersion medium mainly containing water. and (ii) at least (B) a coagulant stored in a different container, wherein (B) the coagulant dissociates a divalent or more metal ion within the dispersion medium, and wherein (B) the coagulant is at least one type selected from citric acid, lactic acid, acetic acid, malic acid, fumaric acid, maleic acid, tartaric acid, gluconic acid, succinic acid, propionic acid, oxalic acid, benzoic acid or salts thereof.

8. The kit for forming the teat pack for lactating livestock according to claim 7, wherein (i) the base or (ii) the coagulant further comprises at least one type selected from (C) a surfactant, (D) a release agent, (E) a water-soluble polymer, (F) water and/or a water-soluble solvent and (G) an antifreezing agent.

9. Use of the composition according to any one of claims 1 to 6 or the kit for forming the teat pack for lactating livestock according to claim 7 or 8 for preventing mastitis in lactating livestock.

## Patentansprüche

1. Zusammensetzung zum Beschichten der gesamten Oberfläche einer Zitze eines milchproduzierenden Nutztiers durch Bilden eines Films, der eng an einer Zitze des milchproduzierenden Nutztiers anhaftet, wobei die Zusammensetzung mindestens umfasst:
einen Polymerlatex (A), wobei der Polymerlatex (A) eine Emulsion ist, die durch Dispergieren eines natürlichen oder eines synthetischen Kautschuks oder eines synthetischen Polymers als Dispersoid in einem hauptsächlich Wasser enthaltenden Dispersionsmedium erhalten wird, und
ein Koagulationsmittel (B), wobei das Koagulationsmittel (B) ein zwei- oder höherwertiges Metallion innerhalb des Dispersionsmediums dissoziiert, wobei das Koagulationsmittel (B) mindestens ein Typ ist, der aus Zitronensäure, Milchsäure, Essigsäure, Apfelsäure, Fumarsäure, Maleinsäure, Weinsäure, Gluconsäure, Bernsteinsäure, Propionsäure, Oxalsäure, Benzoesäure oder Salzen davon ausgewählt ist.

2. Zusammensetzung nach Anspruch 1, wobei das Zitzenpackungsmaterial für ein milchgebendes Nutztier darüber hinaus mindestens einen Typ umfasst, der ausgewählt ist aus einem Tensid (C), einem Trennmittel (D), einem wasserlöslichen Polymer (E), Wasser und/oder einem wasserlöslichen Lösungsmittel (F) und einem Gefrierschutzmittel (G).

3. Zusammensetzung nach Anspruch 2, wobei das Tensid (C) ein anionisches Tensid ist.

4. Zusammensetzung nach Anspruch 2, wobei das Trennmittel (D) eine Verbindung ist, die eine CF₃-, CF₂-, CH₃- oder CH₂-Gruppe aufweist.

5. Zusammensetzung nach Anspruch 2, wobei das wasserlösliche Polymer (E) mindestens ein Typ ist, der ausgewählt ist aus Methylcellulose, Carboxymethylcellulose, Hydroxyethylcellulose, Casein, Polyvinylalkohol, Polyvinylpyrrolidon, Polyvinylmethylether, Polyacrylsäure, Polymethacrylsäure, Polyacrylamid, Polyethylenoxid und Polyethylenimid.

6. Zusammensetzung nach Anspruch 2, wobei das Gefrierschutzmittel (G) mindestens ein Typ ist, der aus der Gruppe ausgewählt ist, die aus Ethylenglykol, Propylenglykol, Glycerin, Alkoholen, Kaliumacetat und Calciumchlorid besteht.

7. Kit zur Bildung einer Packung, die an der Zitze eines milchproduzierenden Nutztiers anhaftet,
umfassend (i) eine Basis, die mindestens einen Polymerlatex (A), der in einem vorbestimmten Behälter (3) untergebracht ist, wobei der Polymerlatex (A) eine Emulsion ist, die durch Dispergieren eines natürlichen oder eines synthetischen Kautschuks oder eines synthetischen Polymers als Dispersoid in einem Dispersionsmedium, das hauptsächlich Wasser enthält, erhalten wird, und (ii) mindestens ein Koagulationsmittel (B) aufweist, das in einem anderen Behälter untergebracht ist, wobei das Koagulationsmittel (B) ein zwei- oder höherwertiges Metallion in dem Dispersionsmedium dissoziiert, und wobei das Koagulationsmittel (B) mindestens ein Typ ist, der ausgewählt ist aus Zitronensäure, Milchsäure, Essigsäure, Apfelsäure, Fumarsäure, Maleinsäure, Weinsäure, Gluconsäure, Bernsteinsäure, Propionsäure, Oxalsäure, Benzoesäure oder Salzen davon.

8. Kit zur Bildung der Zitzenpackung für ein milchgebendes Nutztier nach Anspruch 7, wobei (i) die Basis oder (ii) das Koagulationsmittel darüber hinaus mindestens einen Typ aufweist, der ausgewählt ist aus einem Tensid (C), einem Trennmittel (D), einem wasserlöslichen Polymer (E), Wasser und/oder einem wasserlöslichen Lösungsmittel (F) und einem Gefrierschutzmittel (G).

9. Verwendung der Zusammensetzung nach einem der Ansprüche 1 bis 6 oder des Kits zur Bildung der Zitzenpackung für ein milchgebendes Nutztier nach Anspruch 7 oder 8 zur Verhinderung von Mastitis bei einem milchgebendem Nutztier.

## Revendications

1. Composition pour revêtir la surface entière d'un trayon d'un animal de bétail producteur de lait en formant un film qui adhère étroitement à un trayon de l'animal de bétail producteur de lait, cette composition comprenant au moins
un latex polymère (A), le latex polymère (A) étant est une émulsion obtenue par dispersion d'un caoutchouc naturel ou synthétique, ou d'un polymère synthétique sous forme d'un dispersoïde dans un milieu de dispersion contenant principalement de l'eau et
un coagulant (B), le coagulant (B) dissociant un ion métallique divalent ou plus dans le milieu de dispersion, le coagulant (B) étant au moins un type choisi parmi l'acide citrique, l'acide lactique, l'acide acétique, l'acide malique, l'acide fumarique, l'acide maléique, l'acide tartrique, l'acide gluconique, l'acide succinique, l'acide propionique, l'acide oxalique, l'acide benzoïque ou leurs sels.

2. Composition selon la revendication 1, dans laquelle le matériau de pelliculage des trayons pour l'animal de bétail producteur de lait comprend en outre au moins un type choisi parmi un tensioactif (C), un agent de séparation (D), un polymère hydrosoluble (E), de l'eau et/ou un solvant hydrosoluble (F) et un agent antigel (G).

3. Composition selon la revendication 2, dans laquelle le tensioactif (C) est un tensioactif anionique.

4. Composition selon la revendication 2, dans laquelle l'agent de séparation (D) est un composé comprenant un groupe -CF₃, -CF₂, -CH₃, ou -CH₂.

5. Composition selon la revendication 2, dans laquelle le polymère hydrosoluble (E) est au moins un type choisi parmi la méthylcellulose, la carboxyméthylcellulose, l'hydroxyéthylcellulose, la caséine, l'alcool polyvinylique, la polyvinylpyrrolidone, le polyvinylméthyléther, l'acide polyacrylique, l'acide polyméthacrylique, le polyacrylamide, l'oxyde de polyéthylène et le polyéthylèneimide.

6. Composition selon la revendication 2, dans laquelle l'agent antigel (G) est au moins un type choisi dans le groupe constitué par l'éthylène glycol, le propylène glycol, la glycérine, les alcools, l'acétate de potassium et le chlorure de calcium.

7. Kit pour former un pelliculage qui adhère au trayon de l'animal de bétail producteur de lait,
comprenant (i) une base comprenant au moins un latex polymère (A) logé dans un récipient prédéterminé (3), dans lequel le latex polymère (A) est une émulsion obtenue en dispersant un caoutchouc naturel ou synthétique, ou un polymère synthétique sous la forme d'un dispersoïde dans un milieu de dispersion contenant principalement de l'eau, et (ii) au moins un coagulant (B) qui est stocké dans un récipient différent, le coagulant (B) dissociant un ion métallique divalent ou plus dans le milieu de dispersion, et le coagulant (B) étant au moins un type choisi parmi l'acide citrique, l'acide lactique, l'acide acétique, l'acide malique, l'acide fumarique, l'acide maléique, l'acide tartrique, l'acide gluconique, l'acide succinique, l'acide propionique, l'acide oxalique, l'acide benzoïque ou leurs sels.

8. Kit pour former le pelliculage de trayon pour un animal de bétail producteur de lait selon la revendication 7, dans lequel
(i) la base ou (ii) le coagulant comprend en outre au moins un type choisi parmi un tensioactif (C), un agent de séparation (D), un polymère hydrosoluble (E), de l'eau et/ou un solvant hydrosoluble (F) et un agent antigel (G).

9. Utilisation de la composition selon l'une quelconque des revendications 1 à 6 ou du kit de formation du pelliculage de trayon pour un animal de bétail producteur de lait selon la revendication 7 ou 8 pour prévenir les mastites chez un animal de bétail producteur de lait.
